(19) Europäisches Patentamt
European Patent Office
Office européen des brevets

(11) Veröffentlichungsnummer: **0 059 390**
**B1**

(12) # EUROPÄISCHE PATENTSCHRIFT

(45) Veröffentlichungstag der Patentschrift: **29.05.85**

(21) Anmeldenummer: **82101272.1**

(22) Anmeldetag: **19.02.82**

(51) Int. Cl.[4]: **C 07 D 487/04,** C 07 D 487/14, C 07 D 495/22, A 61 K 31/55 // (C07D487/04, 243:00, 235:00)

(54) **Imidazodiazepine, Zwischenprodukte und Verfahren für ihre Herstellung, sowie diese enthaltende Arzneimittel.**

(30) Priorität: **27.02.81 CH 1341/81**

(43) Veröffentlichungstag der Anmeldung: **08.09.82 Patentblatt 82/36**

(45) Bekanntmachung des Hinweises auf die Patenterteilung: **29.05.85 Patentblatt 85/22**

(84) Benannte Vertragsstaaten: **BE CH DE FR GB IT LI NL SE**

(56) Entgegenhaltungen:
**EP - A - 0 027 214**
**DE - A - 2 809 591**
**DE - A - 2 813 549**

**Patent Abstracts of Japan, Band 2, Nr. 131, 31. Oktober 1978, Seite 2735C78**

(73) Patentinhaber: **F. HOFFMANN-LA ROCHE & CO. Aktiengesellschaft, CH-4002 Basel (CH)**

(72) Erfinder: **Hunkeler, Walter, Dr., Im Stigler 32, CH-4465 Magden (CH)**
Erfinder: **Kyburz, Emilio, Dr., Unterer Rebbergweg 127, CH-4153 Reinach (CH)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

**EP 0 059 390 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft Imidazodiazepine. Im speziellen betrifft sie Imidazodiazepine der allgemeinen Formel

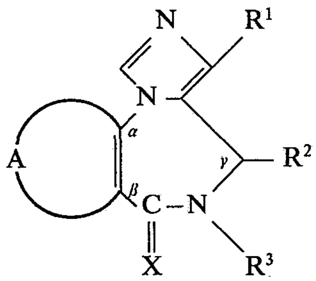

(I)

worin A zusammen mit den beiden mit $\alpha$ und $\beta$ bezeichneten Kohlenstoffatomen die Gruppe

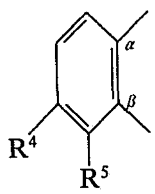

oder

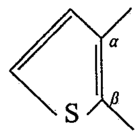

(a) (b)

$R^1$ Wasserstoff, niederes Alkyl, niederes Alkoxymethyl, Halogen oder Nitro, $R^4$ Wasserstoff, Trifluormethyl oder Halogen, $R^5$ Wasserstoff, Trifluormethyl, Halogen oder niederes Alkyl und X ein Sauerstoff- oder Schwefelatom bedeuten, und entweder $R^2$ Wasserstoff und $R^3$ niederes Alkyl bedeuten, oder $R^2$ und $R^3$ zusammen Trimethylen oder Propenylen bedeuten und das mit $\gamma$ bezeichnete Kohlenstoffatom die (S)- oder (R,S)-Konfiguration aufweist,
und pharmazeutisch annehmbare Säureadditionssalze davon.

Diese Verbindungen sind neu und zeichnen sich durch wertvolle pharmakodynamische Eigenschaften aus; sie können bei der Bekämpfung bzw. Verhütung von Krankheiten verwendet werden.

Gegenstand der vorliegenden Erfindung sind Verbindungen der allgemeinen Formel I pharmazeutisch annehmbare Säureadditionssalze davon als solche und als pharmazeutische Wirkstoffe, die Herstellung dieser Verbindungen und Zwischenprodukte für die Herstellung dieser Verbindungen, ferner Arzneimittel, enthaltend eine Verbindung der allgemeinen Formel I oder ein pharmazeutisch annehmbares Säureadditionssalz davon, und die Herstellung solcher Arzneimittel.

Der Ausdruck »nieders Alkyl« bezeichnet gesättigte Kohlenwasserstoffreste, welche geradkettig oder verzweigt sein können, mit höchstens 7, vorzugsweise höchstens 4 Kohlenstoffatomen, wie Methyl, Äthyl, n-Propyl, Isopropyl, n-Butyl, s-Butyl, t-Butyl und dergleichen. Der Ausdruck »niederes Alkoxymethyl« umfaßt Reste wie Methoxymethyl, Äthoxymethyl und dergleichen. Der Ausdruck »Halogen« bedeutet die vier Formen Fluor, Chlor, Brom und Jod.

$R^1$ bedeutet vorzugsweise Wasserstoff, Methyl, Methoxymethyl, Halogen oder Nitro. Wenn $R^2$ Wasserstoff bedeutet, so ist die bevorzugte Bedeutungsmöglichkeit von $R^3$ Methyl. Wenn $R^2$ und $R^3$ zusammen Trimethylen bedeutet, so weist das in Formel I mit $\gamma$ bezeichnete Kohlenstoffatom vorzugsweise die (S)-Konfiguration auf.

Bedeutet das Symbol A die eingangs dargestellte Gruppe (a), so bedeuten $R^4$ vorzugsweise Wasserstoff, Chlor oder Fluor und $R^5$ vorzugsweise Wasserstoff oder Chlor, wobei vorzugsweise mindestens eines von $R^4$ und $R^5$ Wasserstoff bedeutet.

Im Rahmen der vorliegenden Erfindung besonders bevorzugte, von der allgemeinen Formel I umfaßte Verbindungen sind:

3,7-Dichlor-4,5-dihydro-5-methyl-6H-imidazo[1,5-a] -[1,4]benzodiazepin-6-on,
(R,S)-1,8-Dichlor-11,13a-dihydro-9H-imidazo[1,5-a]-pyrrolo[2,1-c] [1,4]benzodiazepin-9-on,
3-Chlor-4,5-dihydro-5-methyl-6H-imidazo[1,5-a] [1,4]-benzodiazepin-6-on und
7-Chlor-4,5-dihydro-3-(methoxymethyl)-5-methyl-6H-imidazo[1,5-a] [1,4]benzodiazepin-6-on.

Weitere im Rahmen der vorliegenden Erfindung bevorzugte, von der allgemeinen Formel I umfaßte Verbindungen sind:

(S)-1-Chlor-10,11,12,12a-tetrahydro-8H-imidazo[5,1-c]-pyrrolo[1,2-a]thieno[3,2-e] [1,4]-

diazepin-8-on,
3-Chlor-8-fluor-4,5-dihydro-5-methyl-6H-imidazo[1,5-a] [1,4]benzodiazepin-6-on,
4,5-Dihydro-3-(methoxymethyl)-5-methyl-6H-imidazo[1,5-a] [1,4]benzodiazepin-6-on,
4,5-Dihydro-3-jod-5-methyl-6H-imidazo[1,5-a] [1,4]benzodiazepin-6-on,
4,5-Dihydro-5-methyl-3-nitro-6H-imidazo[1,5-a] [1,4]benzodiazepin-6-on,
3-Brom-4,5-dihydro-5-methyl-6H-imidazo[1,5-a] [1,4]benzodiazepin-6-on,
7-Chlor-4,5-dihydro-5-methyl-6H-imidazo[1,5-a] [1,4]benzodiazepin-6-on,
3-Brom-8-fluor-4,5-dihydro-5-methyl-6H-imidazo[1,5-a] [1,4]benzodiazepin-6-on,
3-Chlor-4,5-dihydro-5-methyl-6H-imidazo[1,5-a] [1,4]benzodiazepin-6-thion,
(S)-1-Chlor-11,12,13,13a-tetrahydro-9H-imidazo[1,5-a]pyrrolo[2,1-c] [1,4]benzodiazepin-9-on,
(S)-1-Brom-11,12,13,13a-tetrahydro-9H-imidazo[1,5-a]pyrrolo[2,1-c] [1,4]benzodiazepin-9-on und
(R,S)-8-Chlor-11,13a-dihydro-9H-imidazo[1,5-a]pyrrolo[2,1-c] [1,4]benzodiazepin-9-on.

Die Imidazodiazepine der allgemeinen Formel I und ihre pharmazeutisch annehmbaren Säureadditionssalze können erfindungsgemäß hergestellt werden, indem man

a) eine Verbindung der allgemeinen Formel

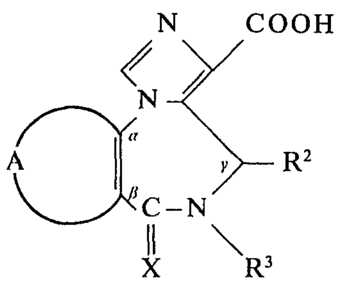

(II)

worin A, X, $R^2$ und $R^3$ obige Bedeutung besitzen, decarboxyliert, oder

b) eine Verbindung der allgemeinen Formel

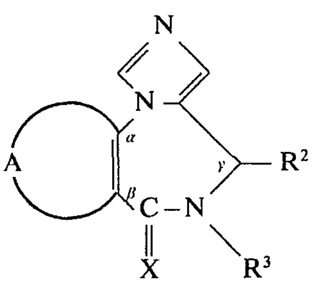

(Ia)

worin A, X, $R^2$ und $R^3$ obige Bedeutung besitzen, am Imidazolring halogeniert, oder

c) in einer Aminoverbindung der allgemeinen Formel

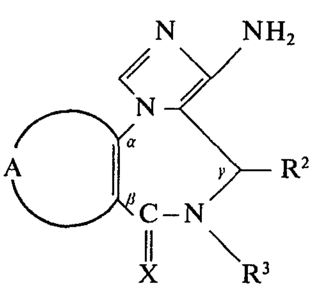

(III)

worin A, X, $R^2$ und $R^3$ obige Bedeutung besitzen, die Aminogruppe durch eine Nitrogruppe oder durch ein Halogenatom ersetzt, oder

d) in einer Aminoverbindung der obigen allgemeinen Formel III die Aminogruppe zur Nitrogruppe oxidiert, oder

e) eine Verbindung der allgemeinen Formel

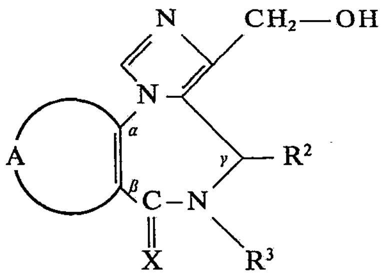

(X)

bzw.

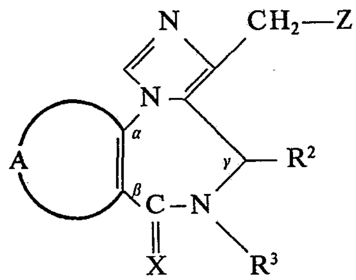

(IVa)

worin A, X, $R^2$ und $R^3$ obige Bedeutung besitzen und Z eine Abgangsgruppe bedeutet, mit einem einen niederen Alkylrest liefernden Alkylierungsmittel bzw. mit einem niederen Alkohol veräthert, oder

f) in einer Verbindung der allgemeinen Formel

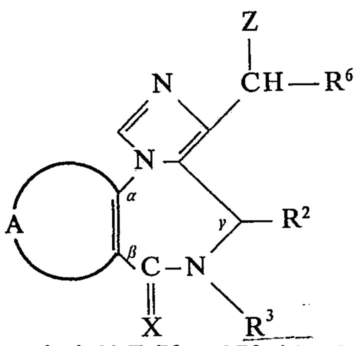

(IV)

worin A, X, Z, $R^2$ und $R^3$ obige Bedeutung besitzen und $R^6$ Wasserstoff oder $(C_{1-6})$-Alkyl bedeutet, die mit Z bezeichnete Abgangsgruppe unter reduktiven Bedingungen abspaltet, oder

g) in einer Verbindung der allgemeinen Formel

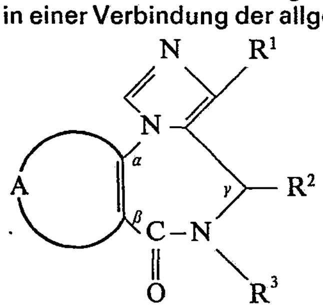

(Ib)

worin A, $R^1$, $R^2$ und $R^3$ obige Bedeutung besitzen, die Carbonylgruppe in die Thiocarbonylgruppe überführt, und

h) erwünschtenfalls eine erhaltene Verbindung der allgemeinen Formel I in ein pharmazeutisch annehmbares Säureadditionssalz überführt.

Gemäß Verfahrensvariante a) können Verbindungen der allgemeinen Formel I, worin $R^1$ Wasserstoff bedeutet, durch Decarboxylierung von Carbonsäuren der allgemeinen Formel II hergestellt werden. Diese Reaktion erfolgt zweckmäßigerweise durch trockenes Erhitzen der gegebenenfalls rohen Carbonsäure der allgemeinen Formel II auf Temperaturen von etwa 150°C bis etwa 400°C, wobei die Reaktionstemperatur vom Schmelzpunkt der jeweils eingesetzten Verbindung der Formel II abhängt.

Gemäß Verfahrensvariante b) können Verbindungen der allgemeinen Formel I, worin $R^1$ Halogen bedeutet, durch Halogenierung von Verbindungen der allgemeinen Formel Ia hergestellt werden. Geeignete Halogenierungsmittel sind beispielsweise N-Chlorsuccinimid, N-Bromsuccinimid, N-Chloracetamid, N-Bromacetamid und elementares Jod. Als Lösungsmittel verwendet man zweckmäßigerweise inerte organische Lösungsmittel, beispielsweise halogenierte Kohlenwasserstoffe, wie Methylenchlorid, 1,2-Dichloräthan, Chloroform und dergleichen, Dimethylformamid, Dimethylacetamid, Acetonitril, Äther, wie Diäthyläther, Tetrahydrofuran, Dioxan und dergleichen, usw. Die Halogenierung kann in einem Temperaturbereich von etwa 0° C bis Siedetemperatur der Reaktionsmischung durchgeführt werden, wobei ein Bereich von etwa Raumtemperatur bis etwa 100° C bevorzugt wird.

Gemäß Verfahrensvariante c) können Verbindungen der allgemeinen Formel I, worin $R^1$ Halogen oder Nitro bedeutet, hergesellt werden, indem man in einer Verbindung der allgemeinen Formel III die Aminogruppe durch ein Halogenatom oder die Nitrogruppe ersetzt. Zweckmäßigerweise geht man dabei so vor, daß man die Aminoverbindung der Formel III in ein entsprechendes Diazoniumsalz überführt und dieses, gegebenenfalls ohne vorgängige Isolierung, mit einem Nitrit, wie Natriumnitrit, oder mit einem Halogenid, z. B. mit einem Chlorid oder Bromid, in Gegenwart eines Kupfer(I)salzes umsetzt. Für die Herstellung der entsprechenden Jodide ist die Anwesenheit eines Kupfer(I)salzes nicht erforderlich. Entsprechende Fluoride werden zweckmäßigerweise über die entsprechenden Diazonium-tetrafluoroborate hergestellt, beispielsweise durch Bestrahlen mit UV-Licht. Diese Reaktionen werden in wäßrigen Lösungen bei Temperaturen von etwa −10° C bis etwa Raumtemperatur durchgeführt.

Gemäß Verfahrensvariante d) können Verbindungen der allgemeinen Formel I, worin $R^1$ Nitro bedeutet, auch hergestellt werden, indem man eine Aminoverbindung der Formel III oxidiert. Als Oxidationsmittel eignen sich beispielsweise Persäuren, wie Peressigsäure, Trifluorperessigsäure, m-Chlorperbenzoesäure und Perbenzoesäure, und dergleichen. Als Lösungsmittel kommen, je nach eingesetztem Oxidationsmittel, Carbonsäuren, wie Essigsäure etc., halogenierte Kohlenwasserstoffe, wie Methylenchlorid, Chloroform, 1,2-Dichloräthan etc., oder dergleichen in Frage. In der Regel arbeitet man bei Temperaturen von etwa 0° C bis etwa Raumtemperatur.

Gemäß Verfahrensvariante e) können Verbindungen der allgemeinen Formel I, worin $R^1$ niederes Alkoxymethyl bedeutet, hergestellt werden, indem man einen Alkohol der allgemeinen Formel X mit einem einen niederen Alkylrest liefernden Alkylierungsmittel, oder eine Verbindung der allgemeinen Formel IVa mit einem niederen Alkohol veräthert. Diese Reaktion erfolgt in einem inerten organischen Lösungsmittel, wie Dimethylformamid, Hexamethylphosphorsäuretriamid, Dimethylsulfoxid, Tetrahydrofuran oder irgend einem anderen geeigneten organischen Lösungsmittel, und in Gegenwart einer Base, die genügend stark basisch ist, um aus dem Alkohol der Formel X bzw. aus dem niederen Alkohol das entsprechende Alkoholat zu bilden. Als Basen eignen sich z. B. Alkalimetallhydride, wie Natriumhydrid, Alkalimetalle, wie Natrium, und Alkalimetallamide, wie Lithumamid und Lithiumdiisopropylamid. Geeignete Alylierungsmittel sind z. B. Alkylhalogenide, wie Methyljodid, Äthyljodid und Äthylbromid, und Dialkylsulfate, wie Dimethylsulfat und Diäthylsulfat. Die Reaktionstemperatur liegt zweckmäßigerweise zwischen etwa 0° C und etwa 50° C.

Gemäß Verfahrensvariante f) können Verbindungen der allgemeinen Formel I, worin $R^1$ niederes Alkyl bedeutet, hergestellt werden, indem man in einer Verbindung der allgemeinen Formel IV die mit Z bezeichnete Abgangsgruppe unter reduktiven Bedingungen abspaltet. Diese Reaktion erfolgt nach an sich bekannten Methoden, wobei die Wahl der geeigneten Abgangsgruppe Z, sowie die Ermittlung der für die Abspaltung geeigneten Reaktionsbedingungen, unter welchen andere im Molekül enthaltene Strukturelemente nicht angegriffen werden dürfen, dem Fachmann keinerlei Schwierigkeiten bereiten. Für den vorliegenden Verfahrensaspekt besonders geeignete Abgangsgruppen sind beispielsweise Halogenatome, wie Chlor, Brom und Jod, welche sich unter hydrogenolytischen Bedingungen leicht abspalten lassen, beispielsweise durch Behandeln mit elementarem Wasserstoff in Gegenwart eines geeigneten Katalysators (z. B. Palladium/Kohle, Raney-Nickel etc.) in einem inerten organischen Lösungsmittel. Geeignete Lösungsmittel sind beispielsweise Alkohole, wie Methanol, Äthanol und Isopropanol, Äther, wie Diäthyläther, Tetrahydrofuran, Dioxan und Dimethoxyäthan, und dergleichen. Je nach Reaktivität des eingesetzten Katalysators arbeitet man bei Drucken von etwa Normaldruck bis etwa 300 bar und bei Temperaturen von etwa Raumtemperatur bis etwa 150° C.

Gemäß Verfahrensvariante g) können Verbindungen der allgemeinen Formel Ib in entsprechende Verbindungen der Formel I, worin X ein Schwefelatom bedeutet, übergeführt werden, und zwar durch Behandeln mit einem Schwefelungsmittel, was in an sich bekannter Weise erfolgen kann. Beispielsweise kann man als Schwefelungsmittel Phosphorpentasulfid verwenden, wobei dieses vorzugsweise im Überschuß eingesetzt wird und die Reaktion vorteilhafterweise in einem inerten organischen Lösungsmittel, wie Dioxan, Methylenchlorid oder dergleichen, in Gegenwart von Triäthylamin bei einer Temperatur von etwa 50° C bis zur Rückflußtemperatur des Reaktionsgemisches erfolgt. Als Schwefelungsmittel eignen sich indessen auch Verbindungen wie 2,4-Bis(p-methoxyphenyl)-1,3,2,4-dithiaphosphetan-2,4-disulfid; derartige Schwefelungsmittel werden ungefähr in der berechneten Menge eingesetzt, und die Reaktion erfolgt in Gegenwart eines inerten Lösungsmittels, wie z. B. Toluol und Xylol, zweckmäßigerweise bei Rückflußtemperatur des Reaktionsgemisches oder in Hexamethyl-phosphorsäuretriamid zwischen etwa 60 und 10° C.

Gemäß Verfahrensvariante h) können Verbindungen der allgemeinen Formel I erfindungsgemäß in pharmazeutisch annehmbare Säureadditionssalze übergeführt werden. Die Herstellung von solchen pharmazeutisch annehmbaren Säureadditionssalze erfolgt nach allgemein üblichen Methoden. Es kommen sowohl Salze mit anorganischen als auch Salze mit organischen Säuren in Betracht, beispielsweise Hydrochloride, Hydrobromide, Sulfate, Methansulfonate, p-Toluolsulfonate, Oxalate und dergleichen.

Die als Ausgangsstoffe verwendeten Verbindungen der allgemeinen Formel II können ausgehend von Verbindungen der Formel

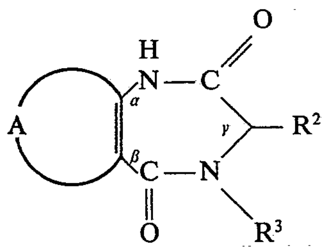

(V)

worin A, $R^2$ und $R^3$ obige Bedeutung besitzen,
hergestellt werden, und zwar gemäß nachfolgendem Formelschema 1, worin A, $R^2$ und $R^3$ obige Bedeutung besitzen, Y eine Abgangsgruppe und $R^7$ niederes Akyl bedeuten:

Formelschema 1

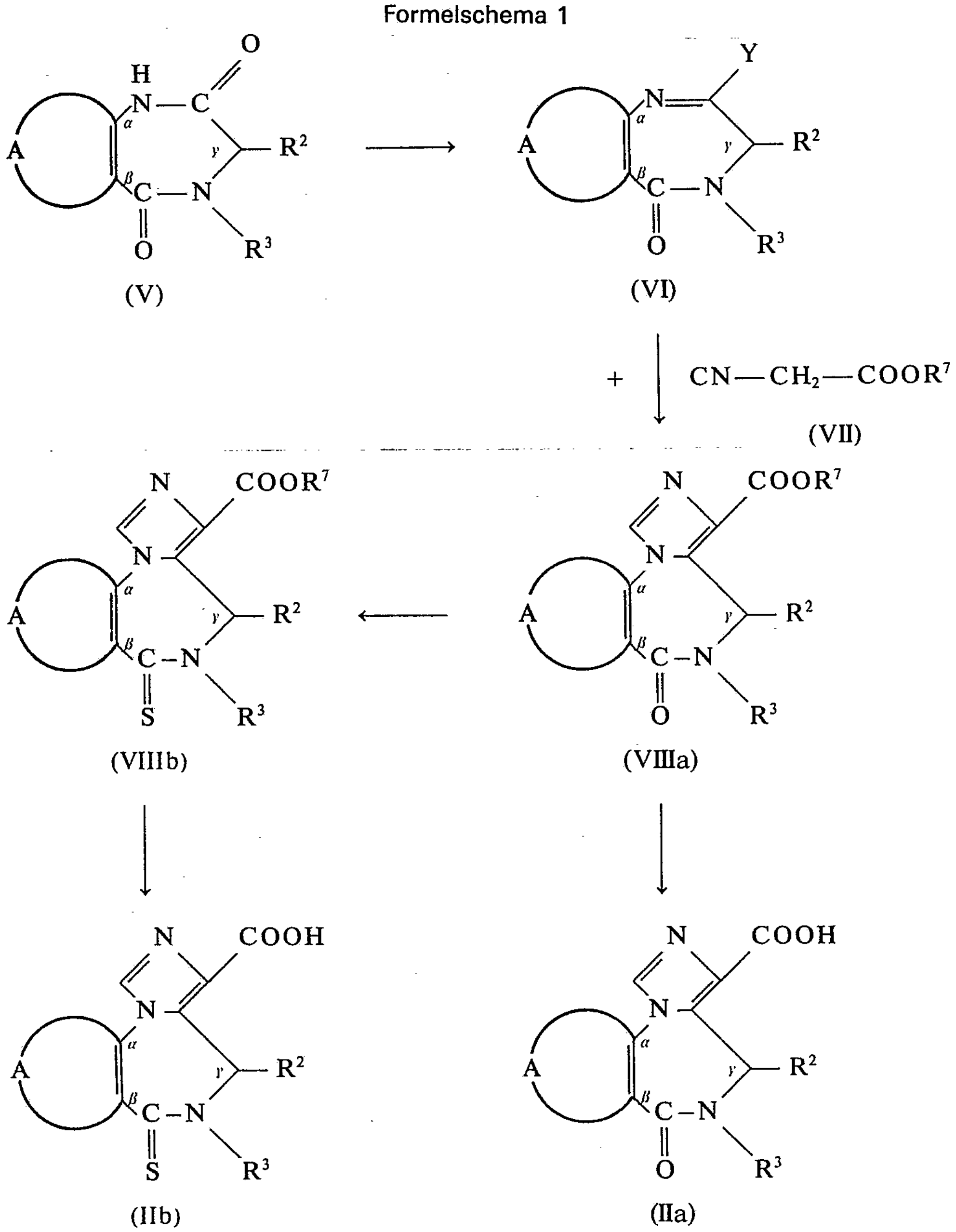


(V) (VI) (VIIIb) (VIIIa) (IIb) (IIa)

Die in Formel VI mit Y bezeichnete Abgangsgruppe ist beispielsweise eine leicht abspaltbare Phosphinylgruppe, z. B. eine Gruppe der Formel

$$-O\overset{\overset{O}{\|}}{P}(OR^8)_2 \quad \text{oder} \quad -O\overset{\overset{O}{\|}}{P}(NR^9R^{10})_2$$

worin $R^8$ niederes Alkyl und $R^9$ und $R^{10}$ je niederes Alkyl, Allyl, Phenyl oder substituiertes Phenyl oder zusammen mit dem Stickstoffatom einen unsubstituierten oder substituierten heterocyclischen Ring mit 3 bis 8 Gliedern (wie Morpholin) bedeuten,
ein Halogenatom, eine Alkylthiogruppe, eine Aralkylthiogruppe, eine N-Nitrosoalkylaminogruppe, eine Alkoxygruppe, eine Mercaptogruppe und dergleichen (wenn Y eine Mercaptogruppe bedeutet, so handelt es sich bei der entsprechenden Verbindung der Formel VI um die Iminothiol-Form des entsprechenden Thiolactams). Die Verbindungen der Formel VI können nach an sich bekannten Methoden aus Verbindungen der Formel V hergestellt werden; vgl. z. B. die belgischen Patentschriften Nr. 802 233, 833 249 und 865 653, die US-Patentschrift Nr. 3 681 341 und J. Org. Chemistry 29, 231 (1964).

Verschiedene der weiter unten folgenden Beispiele enthalten detaillierte Angaben betreffend die Herstellung von Verbindungen der allgemeinen Formel VI aus Verbindungen der allgemeinen Formel V.

Die Reaktion einer Verbindung der Formel VI mit einem Isocyanessigsäurealkylester der Formel VII zu einer Verbindung der allgemeinen Formel VIIIa erfolgt in einem inerten Lösungsmittel, wie Dimethylformamid, Hexamethylphosphorsäuretriamid, Dimethylsulfoxid, Tetrahydrofuran oder irgend einem anderen geeigneten organischen Lösungsmittel, und in Gegenwart einer Base, die genügend stark basisch ist, um das Anion des Isocyanessigsäurealkylesters der Formel VII zu bilden. Als Basen eignen sich Alkalimetallalkoxide, wie Natriummethoxid oder Kalium-t-butoxid, Alkalimetallhydride, wie Natriumhydrid, Alkalimetallamide, wie Lithiumamid oder Lithiumdiisopropylamid, tertiäre Amine, wie Triäthylamin, und dergleichen. Die Reaktionstemperatur liegt zweckmäßigerweise zwischen etwa $-40°$ C und etwa Raumtemperatur.

Die Verbindungen der allgemeinen Formel VIIIb können in Analogie zur weiter oben beschriebenen Verfahrensvariante g) aus Verbindungen der allgemeinen Formel VIIIa hergestellt werden.

Die Hydrolyse von Carbonsäureestern der Formeln VIIIa und VIIIb zu den Carbonsäuren der Formel II, d. h. IIa bzw. IIb, erfolgt nach an sich bekannten und jedem Fachmann geläufigen Methoden. Zweckmäßigerweise erfolgt die Hydrolyse unter basischen Bedingungen, beispielsweise unter Verwendung von wäßrigen, basischen Lösungen (z. B. wäßrige Natronlauge, wäßrige Kaliumcarbonatlösung oder dergleichen), gegebenenfalls in Gegenwart eines Lösungsvermittlers (z. B. Methanol, Äthanol, Tetrahydrofuran, Dioxan oder dergleichen). Handelt es sich bei einer Verbindung der Formel VIIIa bzw. VIIIb um einen t-Alkylester, z. B. um einen t-Butylester, so erfolgt die Hydrolyse vorteilhafterweise unter sauren Bedingungen, beispielsweise unter Verwendung von Trifluoressigsäure, wäßrigen Mineralsäuren oder dergleichen.

Die als Ausgangsstoffe verwendeten Verbindungen der allgemeinen Formel V gehören einer an sich bekannten Stoffklasse an; spezifisch nicht vorbeschriebene Vertreter dieser Stoffklasse können in Analogie zu den bekannten Vertretern hergestellte werden. Außerdem enthalten verschiedene der weiter unten folgenden Beispiele detaillierte Angaben betreffend die Herstellung von Verbindungen der allgemeinen Formel V.

Die Verbindungen der allgemeinen Formel III können aus Carbonsäureaziden der allgemeinen Formel

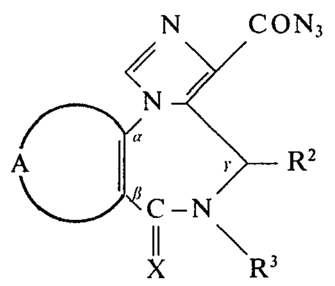

(IX)

worin A, X, $R^2$ und $R^3$ obige Bedeutung besitzen,
leicht hergestellt werden. Beispielsweise kann man ein Carbonsäureazid der Formel IX bei erhöhter Temperatur mit einem Alkohol, z. B mit Methanol, Äthanol, Benzylalkohol oder dergleichen, umsetzen und das erhaltene Urethan hydrolysieren. In einer bevorzugten Ausführungsform verwendet man Benzylalkohol und spaltet das erhaltene Benzylurethan hydrogenolytisch, und zwar nach an sich bekannten und jedem Fachmann geläufigen Methoden.

Die Carbonsäureazide der Formel IX können beispielsweise durch Behandeln eines Carbonsäureesters der Formel VIIIa bzw. VIIIb mit Hydrazin und Umsetzen des erhaltenen Hydrazides mit Natriumnitrit in Gegenwart einer Säure, wie Essigsäure, hergestellt werden.

Die als Ausgangsstoffe verwendeten Verbindungen der allgemeinen Formel IV und X können ausgehend von Verbindungen der allgemeinen Formel VIII, d. h. VIIIa und VIIIb, gemäß nachfolgendem Formelschema 2, worin A, X, Z, $R^2$, $R^3$ und $R^7$ obige Bedeutung besitzen und $R^{61}$ ($C_{1-6}$)-Alkyl bedeutet, hergestellt werden.

Formelschema 2

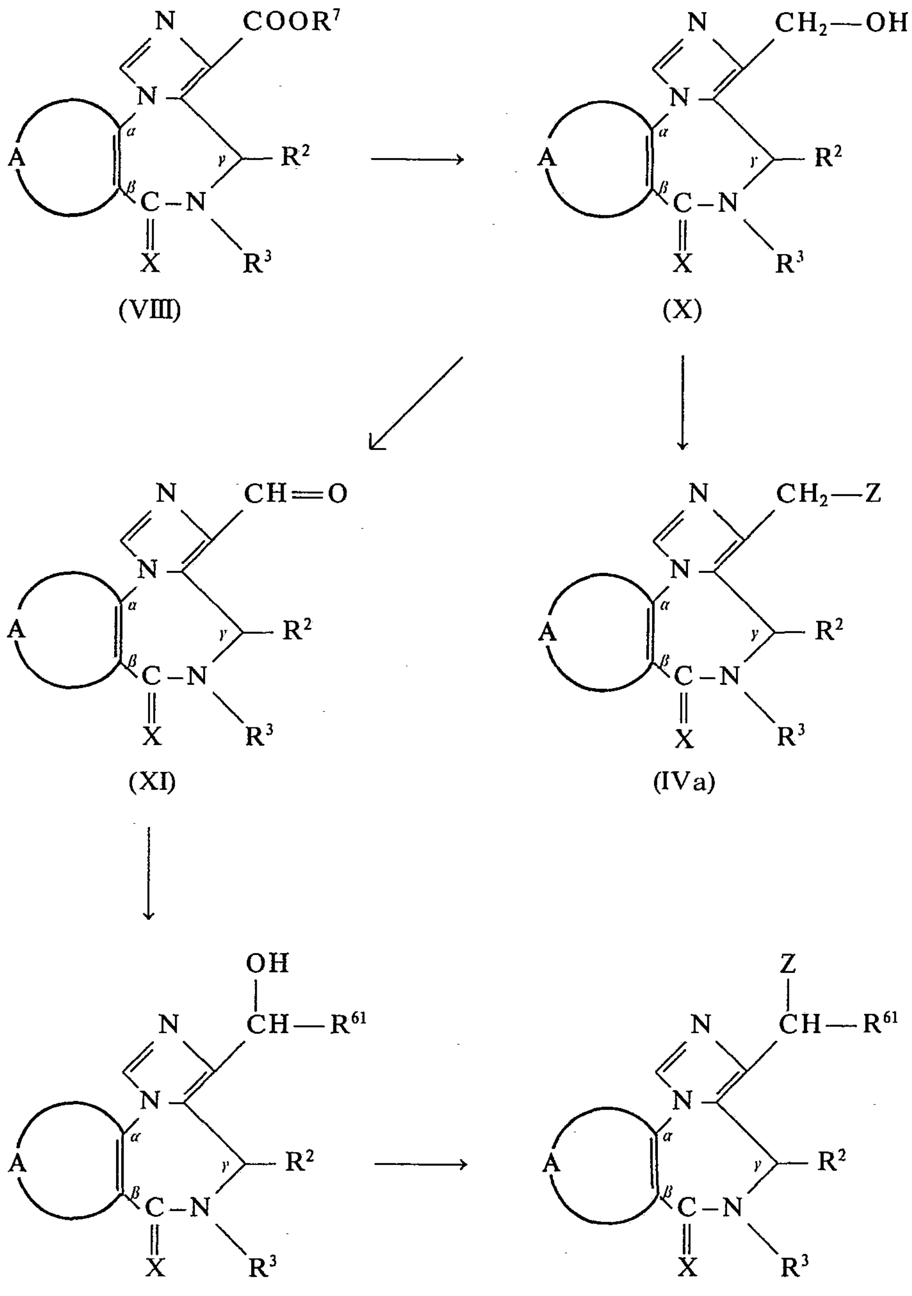

(VIII) (X) (XI) (IVa) (XII) (IVb)

Die Reduktion eines Carbonsäureesters der Formel VIII zu einem Alkohol der Formel X kann mit einem Reduktionsmittel, wie Lithiumborhydrid, in einem inerten organischen Lösungsmittel, wie Diäthyläther, Tetrahydrofuran, Dimethoxyäthan oder dergleichen, durchgeführt werden.

Die Oxidation einer Verbindung der Formel X zu einem Aldehyd der Formel XI erfolgt zweckmäßigerweise mit einem milden Oxidationsmittel, wie Mangandioxid oder dergleichen, in einem inerten organischen Lösungsmittel, wie Methylenchlorid, Chloroform oder dergleichen.

Nach allgemein bekannten und jedem Fachmann geläufigen Methoden kann man einen Aldehyd der allgemeinen Formel XI mit einer den Rest $R^{61}$ liefernden metallorganischen Verbindung umsetzen und erhält somit eine Verbindung der allgemeinen Formel XII. Als metallorganische Verbindungen kommen in erster Linie Grignard-Verbindungen, wie Methyl-magnesiumjodid, Äthyl-magnesiumjodid, Isopropyl-magnesiumjodid, n-Propyl-magnesiumbromid, n-Butyl-magnesiumchlorid und dergleichen, in

Frage. Als Lösungsmittel eignen sich Äther, wie Diäthyläther, Tetrahydrofuran, t-Butyl-methyläther, Mischungen davon, und dergleichen. Zweckmäßigerweise arbeitet man bei der Siedetemperatur des Reaktionsgemisches, man kann aber auch unterhalb davon arbeiten, beispielsweise bei Raumtemperatur.

Die Herstellung von Verbindungen der allgemeinen Formel IV (d. h. IVa und IVb) aus Verbindungen der allgemeinen Formel X bzw. XII erfolgt nach an sich bekannten Methoden. Entsprechende Halogenide können beispielsweise durch Behandeln von Verbindungen der Formel X bzw. XII mit Halogenierungsmitteln, wie Thionylchlorid, Phosphoroxychlorid, Phosphorpentachlorid, Tetrabromkohlenstoff/Triphenylphosphin oder dergleichen, hergestellt werden.

Die als Ausgangsstoffe verwendeten Verbindungen der allgemeinen Formel III und IV sind neu und ebenfalls Gegenstand der vorliegenden Erfindung.

Wie eingangs erwähnt, sind die Verbindungen der allgemeinen Formel I neu und besitzen äußerst wertvolle pharmakodynamische Eigenschaften. Sie wiesen nur eine geringe Toxizität auf, und es hat sich gezeigt, daß sie eine ausgeprägte Affinität zu den zentralen Benzodiazepin-Rezeptoren haben und die zentral dämpfenden, muskelrelaxierenden, ataktischen, blutdrucksenkenden und atemdepressiven Eigenschaften von tranquilisierend wirksamen 1,4-Benzodiazepinen zu antagonisieren vermögen.

Die Affinität von Verbindungen der allgemeinen Formel I zu den zentralen Benzodiazepin-Rezeptoren wurde nach der in Life Science 20, 2101—2110 (1977) und Science 198, 849—851 (1977) beschriebenen Methoden festgestellt. Nach dieser Methode ermittelt man die Hemmung der Bindung von tritiiertem Diazepam an die spezifischen Benzdiazepin-Rezeptoren im cerebralen Cortex durch die jeweiligen Testsubstanzen. Man bezeichnet als $IC_{50}$ (»50% inhibiting concentration«) diejenige Konzentration der jeweiligen Testsubstanz, welche eine 50proz. Hemmung der spezifischen Bindung des tritiierten Diazepams an die spezifischen Benzodiazepin-Rezeptoren im cerebralen Cortex bewirkt.

Eine der typischen Eigenschaften von tranquilisierend wirksamen 1,4-Benzodiazepinen in Versuchstieren ist ihre ausgeprägte anticonvulsive Wirkung, welche beispielsweise im bekannten und allgemein anerkannten Pentetrazol-Test nachgewiesen werden kann. Diese Eigenschaft wurde verwendet, um den nachstehend beschriebenen Test auszuarbeiten, der es erlaubt, Verbindungen zu ermitteln, die die zentralen Eigenschaften von tranquilisierend wirksamen 1,4-Benzodiazepinen zu antogonisieren vermögen.

In diesem Test verabreicht man Mäusen eine Stunde vor dem Pentetrazol (120 mg/kg, i.p.) 5 mg/kg (i.p.) Diazepam (d. h. eine supramaximale Dosis, die im Pentetrazol-Test an mehr als 900 Mäusen alle Versuchstiere vor krampfartigen Anfällen schützte) und 15 Minuten vor dem Pentetrazol die zu testende Verbindung p.o. Die antagonistische Wirksamkeit der untersuchten Verbindungen, d. h. ihr Vermögen, die Wirkung des Diazepams im Pentetrazol-Test aufzuheben, wird ermittelt, indem man die Mäuse auszählt, die in diesem Test krampfartige Anfälle erleiden.

In der nachstehenden Tabelle werden die Resultate dargestellt, welche mit repräsentativen Vertretern der durch die allgemeine Formel I definierten Verbindungsklasse in dem vorstehend geschilderten Versuch erhalten worden sind. Angegeben wird für jede der darin figurierenden Verbindung der $ED_{50}$-Wert. Als $ED_{50}$ bezeichnet man die Menge der jeweiligen Testverbindung in mg/kg (p.o), die bei 50% der Tiere den Diazepam-Effekt in obigem Versuch aufhebt. Außerdem enthält die Tabelle die oben definierten $IC_{50}$-Werte für die darin angegebenen Testverbindungen.

Tabelle

| Verbindung der Formel I, worin<br>A | $R^4$ | $R^5$ | $R^1$ | $R^2$ | $R^3$ | X | Konfiguration | $IC_{50}$ in nM/l | $ED_{50}$ in mg/kg p. o. |
|---|---|---|---|---|---|---|---|---|---|
| (a) | H | Cl | Cl | H | $-CH_3$ | O | — | 3,6 | 0,9 |
| (a) | H | Cl | Cl | $-CH=CH-CH_2-$ | $-CH=CH-CH_2-$ | O | (R/S) | 3,9 | 1,0 |
| (a) | H | H | Cl | H | $-CH_3$ | O | — | 100 | 1,7 |
| (b) | — | — | Cl | $-(CH_2)_3-$ | $-(CH_2)_3-$ | O | (S) | 17 | 0,83 |
| (a) | F | H | Cl | H | $-CH_3$ | O | — | 28 | 2,3 |
| (a) | H | H | J | H | $-CH_3$ | O | — | 23 | 2,5 |
| (a) | H | H | $-NO_2$ | H | $-CH_3$ | O | — | 72 | 4,0 |

Tabelle (Fortsetzung)

| Verbindung der Formel I, worin A | $R^4$ | $R^5$ | $R^1$ | $R^2$ | $R^3$ | X | Konfiguration | $IC_{50}$ in nM/l | $ED_{50}$ in mg/kg p. o. |
|---|---|---|---|---|---|---|---|---|---|
| (a) | H | H | Br | H | $-CH_3$ | O | — | 34 | 4,5 |
| (a) | H | Cl | H | H | $-CH_3$ | O | — | 110 | 6,2 |
| (a) | F | H | Br | H | $-CH_3$ | O | — | 51 | 7,1 |
| (a) | H | H | Cl | H | $-CH_3$ | S | — | 250 | 10,5 |
| (a) | H | H | Cl | $-(CH_2)_3-$ | $-(CH_2)_3-$ | O | (S) | 120 | 8,0 |
| (a) | H | H | Br | $-(CH_2)_3-$ | $-(CH_2)_3-$ | O | (S) | 48 | 9,9 |
| (a) | H | Cl | H | $-CH=CH-CH_2-$ | $-CH=CH-CH_2-$ | O | (R/S) | 240 | 10,9 |
| (a) | H | Cl | $-CH_2OCH_3$ | H | $-CH_3$ | O | — | 30 | 1,4 |
| (a) | H | H | $-CH_2OCH_3$ | H | $-CH_3$ | O | — | 380 | 8,9 |

Wie bereits erwähnt, antogonisieren die Verbindungen der Formel I die zentral dämpfenden, muskelrelaxierenden, ataktischen, blutdrucksenkenden und atemdepressiven Eigenschaften von tranquilisierend wirksamen 1,4-Benzodiazepinen. Letztere stehen in der Therapie in vielfältigem Gebrauch und werden oft in hohen Dosierungen angewandt, so daß die oben erwähnten Wirkungen auch als Nebenwirkungen stark hervortreten können. Die Verbindungen der Formel I können bei Intoxikationen, bei welchen übermäßige Einnahme von tranquilisierend wirksamen 1,4-Benzodiazepinen beteiligt ist, als Antidot verwendet werden. Sie eignen sich auch zur Abkürzung einer durch tranquilisierend wirksame 1,4-Benzodiazepine eingeleiteten Anästhesie in der Chirurgie und in der Geburtshilfe. Bei Neugeborenen kann eine eventuelle Atemdepression, die auf die Gabe von tranquilisierend wirksamen 1,4-Benzodiazepinen an die Mutter zurückgeht, aufgehoben werden. Die Verbindungen der Formel I können auch dazu verwendet werden, bei 1,4-Benzodiazepinen, die in anderen Indikationsgebieten eingesetzt werden, die in einem solchen Fall unerwünschten Wirkungen auf das zentrale Nervensystem zu unterdrücken. Beispiele derartiger, in anderen Indikationsgebieten einsetzbarer 1,4-Benzodiazepine sind die in den britischen Patentschriften Nr. 1 444 529 und 1 474 305 beschriebenen schistosomizid wirksamen 1,4-Benzodiazepine, wie das (+)-5-(o-Chlorphenyl)-1,3-dihydro-3-methyl-7-nitro-2H-1,4-benzodiazepin-2-on.

Die Verbindungen der allgemeinen Formel I und ihre pharmazeutisch annehmbaren Säureadditionssalze können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Injektionslösungen, erfolgen.

Zur Herstellung von Tabletten, Lacktabletten, Dragées und Hartgelatinekapseln können die Verfahrensprodukte mit pharmazeutisch inerten, anorganischen oder organischen Trägern verarbeitet werden. Als solche Träger kann man für Tabletten, Dragées und Hartgelatinekapseln z. B. Lactose, Maisstärke oder Derivate davon, Talk, Stearinsäure oder deren Salze etc. verwenden. Für Weichgelatinekapseln eignen sich als Träger z. B. pflanzliche Öle, Wachse, Fette, halbfeste und flüssige Polyole etc. Zur Herstellung von Lösungen und Sirupen eignen sich als Träger z. B. Wasser, Polyole, Saccharose, Invertzucker, Glukose und dergleichen. Für Injektionslösungen eignen sich als Träger z. B. Wasser, Alkohole, Polyole, Glyzerin, pflanzliche Öle etc. Für Suppositorien eignen sich als Träger z. B. natürliche oder gehärtete Öle, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süßmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten.

Wie eingangs erwähnt, kann man Verbindungen der allgemeinen Formel I und pharmazeutisch annehmbare Säureadditionssalze davon bei der Bekämpfung bzw. Verhütung von Krankheiten verwenden, insbesondere bei der Antagonisierung der zentral dämpfenden, muskelrelaxierenden, ataktischen, blutdrucksenkenden und atemdepressiven Eigenschaften von tranquilisierend wirksamen 1,4-Benzodiazepinen. Insbesondere kann man Verbindungen der allgemeinen Formel I in Kombination

mit den weiter oben erwähnten schistosomizid wirksamen Verbindungen, z. B. in Kombination mit (+)-5-(o-Chlorphenyl)-1,3-dihydro-3-methyl-7-nitro-2H-1,4-benzodiazepin-2-on bei der Bekämpfung der Schistosomiasis verwenden. Dabei können die Verbindungen der Formel I oder deren pharmazeutisch annehmbare Säureadditionssalze vor, gleichzeitig mit oder nach der Verabreichung bzw. Einnahme von tranquilisierend wirksamen 1,4-Benzodiazepinen verabreicht werden. Wird die Verbindung der Formel I oder ein pharmazeutisch annehmbares Säureadditionssalz davon gleichzeitig mit dem tranquilisierend wirksamen 1,4-Benzodiazepin verabreicht, so kann dies durch Verabreichung als ad-hoc-Kombination oder in Form einer pharmazeutischen Kombination erfolgen, welche eine Verbindung der allgemeinen Formel I oder ein pharmazeutisch annehmbares Säureadditionssalz davon und ein tranquilisierend wirksames 1,4-Benzodiazepin-Derivat enthält; derartige pharmazeutische Kombinationen sind ebenfalls Gegenstand der vorliegenden Erfindung. Die Dosierung der Verbindungen der Formel I und ihrer pharmazeutisch annehmbaren Säureadditionssalze kann innerhalb weiter Grenzen variieren und ist natürlich in jedem einzelnen Fall den individuellen Gegebenheiten anzupassen. Im allgemeinen dürfte eine Tagesdosis von etwa 0,2 bis etwa 500 mg angemessen sein.

Wie eingangs erwähnt, sind Arzneimittel, enthaltend eine Verbindung der allgemeinen Formel I oder ein pharmazeutisch annehmbares Säureaditionssalz davon, ebenfalls Gegenstand der vorliegenden Erfindung, weiterhin auch ein Verfahren zur Herstellung solcher Arzneimittel, welches dadurch gekennzeichnet ist, daß man eine oder mehrere Verbindungen der allgemeinen Formel I oder pharmazeutisch annehmbare Säureadditionssalze davon und gegebenfalls einen oder mehrere andere therapeutisch wertvolle Stoffe in eine galenische Darreichungsform bringt; in diesem Zusammenhang sei nochmals auf die weiter oben erwähnten pharmazeutischen Kombinationen hingewiesen, welche ebenfalls Gegenstand der vorliegenden Erfindung sind. Im speziellen sind pharmazeutische Kombinationen, enthaltend eine Verbindung der allgemeinen Formel I und eine der weiter oben erwähnten schistosomizid wirksamen Verbindungen, insbesondere das (+)-5-(o-Chlorphenyl)-1,3-dihydro-3-methyl-7-nitro-2H-1,4-benzodiazepin-2-on, Gegenstand der vorliegenden Erfindung; derartige Kombinationen eignen sich zur Bekämpfung von Schistosomiasis.

In den nachfolgenden Beispielen, welche die vorliegende Erfindung näher erläutern, ihren Umfang jedoch in keiner Weise beschränken sollen, sind sämtliche Temperaturen in Celsiusgraden angegeben.

## Beispiel 1

a) Man rührt 29,1 g (0,14 Mol) 6-Chlor-isatosäureanhydrid mit 13,12 g (0,14 Mol) Sarcosin in 150 ml Dimethylsulfoxid während 1 Stunde bei 110°. Die erhaltene Lösung wird eingeengt und der Rückstand aus Äthanol umkristallisiert. Man erhält 6-Chlor-3,4-dihydro-4-methyl-2H-1,4-benzodiazepin-2,5(1H)-dion vom Schmelzpunkt 247—238°.

b) Eine Lösung von 10 g (44,5 mMol) 6-Chlor-3,4-dihydro-4-methyl-2H-1,4-benzodiazepin-2,5(1H)-dion in 100 ml Dimethylformamid wird unter einer Argonatmosphäre mit 5,50 g (49 mMol) Kalium-t-butylat versetzt und 20 Minuten gerührt. Man kühlt die erhaltene Lösung auf −30° ab und tropft bei dieser Temperatur 3,45 g (49 mMol) Diäthylchlorphosphat zu. Das Gemisch wird anschließend 10 Minuten bei −20° gerührt.

Inzwischen kühlt man eine Lösung von 5,50 g (40 mMol) Kalium-t-butylat in 10 ml Dimethylformamid in einem Aceton/Trockeneisbad ab und versetzt mit 5,54 g (49 mMol) Isocyanessigsäureäthylester. Die dunkelrote Lösung wird bei −10 bis −20° dem obigen Reaktionsgemisch zugegeben und dieses eine halbe Stunde ohne Kühlung gerührt bevor man es mit 5 ml Eisessig neutralisiert und auf ca. 300 ml Wasser gießt. Die orange Lösung wird dreimal mit Chloroform extrahiert. Die organische Phase wird fünfmal mit Wasser gewaschen, über Magnesiumsulfat getrocknet und eingedampft. Durch Chromatographieren an Kieselgel und Umkristallisieren aus Essigester erhält man Äthyl-7-chlor-5,6-dihydro-5-methyl-6-oxo-4H-imidazo[1,5-a] [1,4]benzodiazepin-3-carboxylat vom Schmelzpunkt 229—230°.

c) Eine Mischung aus 3,11 g (9,72 mMol) Äthyl-7-chlor-5,6-dihydro-5-methyl-6-oxo-4H-imidazo[1,5-a] [1,4]benzodiazepin-3-carboxylat, 10 ml Äthanol, 2 ml Wasser und 400 mg Natriumhydroxid wird während 1 Stunde unter Rückfluß zum Sieden erhitzt. Nach Abdampfen des Äthanols wird der Rückstand mit 10 ml 1 N-Salzsäure versetzt. Das erhaltene farblose Material wird abgenutscht, mit Wasser gewaschen und getrocknet. Man erhält 7-Chlor-5,6-dihydro-5-methyl-6-oxo-4H-imidazo[1,5-a] [1,4]benzodiazepin-3-carbonsäure mit einem Zersetzungspunkt von 283—284°.

d) 2,69 g 7-Chlor-5,6-dihydro-5-methyl-6-oxo-4H-imidazo[1,5-a] [1,4]benzodiazepin-3-carbonsäure werden auf 290° erhitzt. Nach Beendigung der Kohlendioxidentwicklung wird die braune Schmelze abgekühlt und in Chloroform aufgenommen. Diese Lösung wird über eine Kieselgelsäule filtriert und eingedampft. Man erhält 7-Chlor-4,5-dihydro-5-methyl-6H-imidazo[1,5-a] [1,4]benzodiazepin-6-on vom Schmelzpunkt 200—201°.

## Beispiel 2

1,30 g (5,25 mMol) 7-Chlor-4,5-dihydro-5-methyl-6H-imidazo[1,5-a] [1,4]benzodiazepin-6-on werden in 10 ml Dimethylformamid mit 800 mg (6 mMol) N-Chlorsuccinimid während 30 Minuten bei 90°

gerührt. Anschließend gießt man auf Wasser und extrahiert mit Chloroform. Die vereinigten Chloroformauszüge werden mit Wasser gewaschen, über Magnesiumsulfat getrocknet und eingedampft. Durch Säulenchromatographie an Kieselgel erhält man 3,7-Dichlor-4,5-dihydro-5-methyl-6H-imidazo[1,5-a] [1,4]benzodiazepin-6-on vom Schmelzpunkt 211—212°.

Beispiel 3

a) Eine Mischung aus 175 g (0,93 Mol) 3-Amino-2-thiophencarbonsäuremethylester-hydrochlorid, 1,8 l n-Butanol und 77 g Natronlauge werden während 30 Minuten unter Rückfluß zum Sieden erhitzt. Nach Einengen der erhaltenen Suspension wird das resultierende Gemisch aus Natriumsalz der 3-Amino-2-thiophencarbonsäure und Kochsalz direkt für die nächste Stufe eingesetzt. Dazu wird das Gemisch mit 800 ml Wasser, 280 ml konz. Salzsäure und 230 ml Tetrahydrofuran versetzt. Durch diese Mischung leitet man bei 15 bis 25° während 2,5 Stunden Phosgen ein und anschließend während 15 Minuten Luft. Der ausgefallene Festkörper wird abgenutscht, mit Wasser gewaschen und getrocknet. Man erhält 2H-Thieno[3,2-d] [1,3]oxazin-2,4(1H)-dion vom Schmelzpunkt 220—221°.

b) Eine Lösung von 34,3 g (202 mMol) 2H-Thieno[3,2-d] [1,3]oxazin-2,4(1H)-dion und 23,3 g (202 mMol) L-Prolin in 200 ml Dimethylsulfoxid wird während 1 Stunde bei 110° gerührt. Die erhaltene braune Lösung wird auf 2 l Wasser gegossen und über Nacht bei Raumtemperatur gerührt. Das ausgefallene Produkt wird abgenutscht, im Vakuum getrocknet und mit ca. 200 ml siedenden Essigester gewaschen. Man erhält dabei (S)-5a,6,7,8-Tetrahydro-5H-pyrrolo[1,2-a]thieno[3,2-e] [1,4]diazepin-5,10(4)-dion vom Schmelzpunkt 244—247°.

c) Unter einer Argonatmosphäre werden 7 g (31,5 mMol) (S)-5a,6,7,8-Tetrahydro-5H-pyrrolo[1,2-a]thieno[3,2-e] [1,4]diazepin-5,10(4)-dion in 30 ml Dimethylformamid suspendiert und bei −50° mit 3,92 g (35 mMol) Kalium-t-butylat versetzt. Man rührt die Lösung 10 Minuten bei −50°, tropft bei dieser Temperatur 6,0 g (35 mMol) Diäthylchlorphosphat zu und rührt während einer halben Stunde.

Separat werden 3,92 g (35 mMol) Kalium-t-butylat in 7 ml Dimethylformamid gelöst, im Aceton/Trockeneisbad gekühlt und mit 3,95 g 35 mMol) Isocyanessigsäureäthylester versetzt. Die erhaltene orangefarbene Lösung wird bei −50° zum obigen Reaktionsgemisch getropft. Anschließend rührt man noch 10 Minuten bei −50 bis −60°, neutralisiert mit 3,2 ml Essigsäure und gießt auf ca. 250 ml Wasser. Man extrahiert zweimal mit je 200 ml Chloroform, wäscht die vereinigten Chloroform-Phasen fünfmal mit je 300 ml Wasser, trocknet über Magnesiumsulfat und dampft ein. Durch Säulenchromatographie an Kieselgel und anschließendes Umkristallisieren aus Essigester erhält man Äthyl-(S)-10,11,12,12a-tetrahydro-8-oxo-8H-imidazo[5,1-c]pyrrolo[2,1-a]thieno[3,2-e] [1,4]diazepin-1-carboxylat vom Schmelzpunkt 212,5—213°.

d) Eine Mischung aus 6,34 g (20 mMol) Äthyl-(S)-10,11,12,12a-tetrahydro-8-oxo-8H-imidazo[5,1-c]pyrrolo[1,2-a]thieno[3,2-e] [1,4]diazepin-1-carboxylat, 956 mg (23,9 mMol) Natriumhydroxid, 20 ml Äthanol und 4 ml Wasser wird während 30 Minuten unter Rückfluß zum Sieden erhitzt. Nach Abdampfen des Äthanols wird der Rückstand mit 23,9 ml 1 N-Salzsäure versetzt. Das erhaltene weiße Material wird abgenutscht, mit Wasser gewaschen und getrocknet. Man erhält (S)-10,11,12,12a-Tetrahydro-8-oxo-8H-imidazo[5,1-c]pyrrolo[1,2-a]thieno[3,2-e]diazepin-1-carbonsäure vom Schmelzpunkt 259°.

e) 5,38 g (18,6 mMol) (S)-10,11,12,12a-Tetrahydro-8-oxo-8H-imidazo[5,1-c]pyrrolo[1,2-a]thieno[3,2-e]diazepin-1-carbonsäure werden auf 270° erhitzt. Nach Beendigung der Kohlendioxidentwicklung wird die Schmelze in Essigester aufgenommen und während 10 Minuten gerührt. Das feste Material wird abgenutscht und getrocknet. Man erhält (S)-10,11,12,12a-Tetrahydro-8H-imidazo[5,1-c]pyrrolo[1,2-a]thieno[3,2-e] [1,4]diazepin-8-on vom Schmelzpunkt 228—230°.

Beispiel 4

3,0 g (12,2 mMol) (S)-10,11,12,12a-Tetrahydro-8H-imidazo[5,1-c]pyrrolo[1,2-a]thieno[3,2-e] [1,4]diazepin-8-on in 20 ml Dimethylformamid werden mit 1,63 g (12,2 mMol) N-Chlorsuccinimid während 40 Minuten bei 65° gerührt. Die Lösung wird auf Wasser gegossen und mit Chloroform extrahiert. Die organische Phase wird mehrmals mit Wasser gewaschen, über Magnesiumsulfat getrocknet und eingedampft. Durch Säulenchromatographie an Kieselgel erhält man (S)-1-Chlor-10,11,12,12a-tetrahydro-8H-imidazo[5,1-c]pyrrolo[1,2-a]thieno[3,2-e] [1,4]diazepin-8-on vom Schmelzpunkt 180—183°.

Beispiel 5

a) Ein Gemisch aus 4,8 g (24,3 mMol) 6-Chlorisatosäureanhydrid, 2,83 g (25 mMol) (S)-3,4-Dehydroprolin und 20 ml Dimethylsulfoxid wird während 1,25 Stunden bei 100° gerührt. Anschließend gießt man auf 200 ml Wasser und extrahiert dreimal mit Essigester. Die organische Phase wird einmal mit Wasser gewaschen, über Magnesiumsulfat getrocknet und eingedampft. Man chromatographiert den Rückstand an Kieselgel und kristallisiert das erhaltene Material aus Essigester. Man erhält (S)-6-Chlor-3,11a-dihydro-5H-pyrrolo[2,1-c] [1,4]benzodiazepin-5,11(10H)-dion vom Schmelzpunkt 250°.

b) Eine Suspension von 0,67 g (15,4 mMol) Natriumhydrid (55proz. Öldispersion) in 25 ml trockenem

Dimethylformamid versetzt man mit 3,22 g (12,9 mMol) (S)-6-Chlor-3,11a-dihydro-5H-pyrrolo[2,1-c] [1,4]benzodiazepin-5,11(10H)-dion und rührt während 30 Minuten. Anschließend kühlt man auf −35° ab, tropft 2,1 ml (13,4 mMol) Diäthylchlorphosphat zu und rührt noch während ca. 15 Minuten.

Eine Lösung von 1,55 g (14,2 mMol) Kalium-t-butylat in 5,5 ml trockenem Dimethylformamid, wird in einem Aceton/Trockeneisbad abgekühlt, mit 1,8 ml (14,2 mMol) Isocyanessigsäureäthylester versetzt und bei −10° dem obigen Reaktionsgemisch zugetropft. Man entfernt das Kühlbad, neutralisiert bei Raumtemperatur mit Eisessig, gießt auf 150 ml Wasser und extrahiert dreimal mit Chloroform. Die Chloroformauszüge werden einmal mit Wasser gewaschen, über Magnesiumsulfat getrocknet und eingedampft. Man chromatographiert den Rückstand an Kieselgel und kristallisiert das erhaltene Material aus Essigester um. Man erhält Äthyl-(R,S)-8-chlor-11,13a-dihydro-9-oxo-9H-imidazo[1,5-a]pyrrolo[2,1-c] [1,4]benzodiazepin-1-carboxylat vom Schmelzpunkt 204—206°.

c) Ein Gemisch aus 1,1 g (3,2 mMol) Äthyl-(R,S)-8-chlor-11,13a-dihydro-9-oxo-9H-imidazo[1,5-a]pyrrolo[2,1-c] [1,4]benzodiazepin-1-carboxylat und 0,187 g (4,7 mMol) Natriumhydroxid wird mit 5 ml Wasser und 15 ml Äthanol versetzt und während 0,5 Stunden unter Rückfluß zum Sieden erhitzt. Man versetzt mit 4,7 ml 1 N-Salzsäure, dampft bis zur beginnenden Kristallisation ein, verdünnt mit ca. 50 ml Wasser und läßt während 1 Stunde im Eisbad stehen. Das ausgefallene Material wird abgenutscht, mit Wasser gewaschen und getrocknet. Man erhält (R,S)-8-Chlor-11,13a-dihydro-9-oxo-9H-imidazo[1,5-a]pyrrolo[2,1-c] [1,4]benzodiazepin-1-carbonsäure vom Schmelzpunkt 249°.

d) 0,8 g (2,5 mMol) (R,S)-8-Chlor-11,13a-dihydro-9-oxo-9H-imidazo[1,5-a]pyrrolo[2,1-c] [1,4]benzodiazepin-1-carbonsäure werden bis zur Beendigung der Kohlendioxidentwicklung mit dem Bunsenbrenner erhitzt. Anschließend kocht man das Material mit Essigester aus, läßt für kurze Zeit stehen und nutscht den Festkörper ab. Man erhält (R,S)-8-Chlor-11,13a-dihydro-9H-imidazo[1,5-a]pyrrolo[2,1-c] [1,4]benzodiazepin-9-on vom Schmelzpunkt 271—273°.

## Beispiel 6

1,3 g (4,8 mMol) (R,S)-8-Chlor-11,13a-dihydro-9H-imidazo[1,5-a]pyrrolo[2,1-c] [1,4]benzodiazepin-9-on und 0,64 g (4,8 mMol) N-Chlorsuccinimid werden mit 20 ml Dimethylformamid versetzt und während 40 Minuten bei 100° gerührt. Anschließend gießt man auf 80 ml Wasser und extrahiert viermal mit Chloroform. Die Chloroformauszüge werden dreimal mit Wasser gewaschen, über Magnesiumsulfat getrocknet und eingedampft. Der Rückstand wird unter Eluierung mit Essigester/Chloroform (1 : 3) an Kieselgel chromatographiert. Nach Umkristallisieren aus Essigester/Hexan erhält man (R,S)-1,8-Dichlor-11,13a-dihydro-9H-imidazo[1,5-a]pyrrolo[2,1-c] [1,4]benzodiazepin-9-on vom Schmelzpunkt 235—237°.

## Beispiel 7

a) Man löst 24 g (132,5 mMol) 5-Fluorisatosäureanhydrid in 140 ml Dimethylsulfoxid und versetzt mit 11,8 g (132,5 mMol) Sarcosin. Die Lösung wird bis zum Ende der Gasentwicklung bei 100° gerührt (Dauer: ca. 1,5 Stunden) und anschließend auf ca. 1,2 l Wasser gegossen. Nach 10minütigem Rühren kristallisiert ein Festkörper aus. Die Kristalle werden abgenutscht, mit 1 l Wasser gewaschen und getrocknet. Man erhält 7-Fluor-3,4-dihydro-4-methyl-2H-1,4-benzodiazepin-2,5(1H)-dion vom Schmelzpunkt 262—263°.

b) Unter einer Argonatmosphäre versetzt man eine Lösung von 6,5 g (32 mMol) 7-Fluor-3,4-dihydro-4-methyl-2H-1,4-benzodiazepin-2,5(1H)-dion in 30 ml trockenem Dimethylformamid mit 4,3 g (38 mMol) Kalium-t-butylat. Die Temperatur steigt dabei auf 35°. Nach 10 Minuten kühlt man auf −30° und tropft, bei −30° bis −20°, 5,8 g (34 mMol) Diäthylchlorphosphat zu. Die Lösung wird anschließend 10 Minuten bei −20° gerührt.

Separat werden 4 g (35 mMol) Kalium-t-butylat in 10 ml Dimethylformamid gelöst und bei ca. −40° mit 4 g (35 mMol) Isocyanessigsäureäthylester versetzt. Diese Lösung wird bei −10° bis −20° zum obigen Reaktionsgemisch getropft. Man rührt dann während 1 Stunde ohne Kühlung, gibt 3,2 ml Eisessig zu, gießt auf ca. 400 ml Wasser und extrahiert dreimal mit je 150 ml Essigester. Die vereinigten organischen Auszüge werden fünfmal mit je 200 ml Wasser gewaschen, über Magnesiumsulfat getrocknet und eingedampft. Aus dem öligen Rückstand erhält man durch Säulen-Chromatographie an Kieselgel und anschließendes Umkristallisieren aus Essigester/Äther Äthyl-8-fluor-5,6-dihydro-5-methyl-6-oxo-4H-imidazo[1,5-a] [1,4]benzodiazepin-3-carboxylat vom Schmelzpunkt 199—200°.

c) Eine Lösung von 3,03 g (10 mMol) Äthyl-8-fluor-5,6-dihydro-5-methyl-6-oxo-4H-imidazo[1,5-a] [1,4]benzodiazepin-3-carboxylat und 0,44 g (11 mMol) Natriumhydroxid in 20 ml Äthanol und 10 ml Wasser wird während 30 Minuten unter Rückfluß zum Sieden erhitzt. Anschließend versetzt man mit 11 ml 1 N-Salzsäure und engt auf das halbe Volumen ein. Die ausgefallenen Kristalle werden abgenutscht und getrocknet. Man erhält 8-Fluor-5,6-dihydro-5-methyl-6-oxo-4H-imidazo[1,5-a] [1,4]benzodiazepin-3-carbonsäure vom Zersetzungspunkt 279°.

d) 9,2 g 8-Fluor-5,6-dihydro-5-methyl-6-oxo-4H-imidazo[1,5-a] [1,4]benzodiazepin-3-carbonsäure werden auf 290° erhitzt. Nach Beendigung der Kohlendioxidentwicklung löst man das Produkt in Chloroform und versetzt mit Hexan. Die ausgefallenen Kristalle werden abgenutscht und getrocknet. Man erhält 8-Fluor-4,5-dihydro-5-methyl-6H-imidazo[1,5-a] [1,4]benzodiazepin-6-on vom Schmelzpunkt 236°.

Beispiel 8

2,31 g (10 mMol) 8-Fluor-4,5-dihydro-5-methyl-6H-imidazo[1,5-a] [1,4]benzodiazepin-6-on und 1,47 g (11 mMol) N-Chlorsuccinimid werden in Dimethylformamid während 30 Minuten auf 100° erhitzt. Anschließend gießt man auf Wasser und extrahiert mehrmals mit Chloroform. Die vereinigten Chloroformauszüge werden mit Wasser gewaschen, über Magnesiumsulfat getrocknet und eingedampft. Der Rückstand wird aus Essigester umkristallisiert und liefert 3-Chlor-8-fluor-4,5-dihydro-5-methyl-6H-imidazo[1,5-a] [1,4]benzodiazepin-6-on vom Schmelzpunkt 217—218°.

Beispiel 9

Zu 4,62 g (20 mMol) 8-Fluor-4,5-dihydro-5-methyl-6H-imidazo[1,5-a] [1,4]benzodiazepin-6-on in 100 ml Dimethylformamid gibt man 3,58 g N-Bromsuccinimid und rührt während 2 Stunden bei Raumtemperatur. Anschließend gießt man auf 500 ml Wasser und extrahiert mehrmals mit Chloroform. Die vereinigten Chloroformauszüge werden mit Wasser gewaschen, über Magnesiumsulfat getrocknet und eingedampft. Nach Umkristallisieren des Rückstandes aus Essigester erhält man 3-Brom-8-fluor-4,5-dihydro-5-methyl-6H-imidazo[1,5-a] [1,4]benzodiazepin-6-on vom Schmelzpunkt 200°

Beispiel 10

a) Unter einer Argonatmosphäre werden 19,0 g (0,10 Mol) 3,4-Dihydro-4-methyl-2H-1,4-benzodiazepin-2,5(1H)-dion in 100 ml trockenem Dimethylformamid vorgelegt. Man gibt 15,5 g (0,12 Mol) Kalium-t-butylat zu, wobei die Temperatur von 25° auf 39° steigt. Man kühlt auf Raumtemperatur und tropft zwischen 18 bis 22° 18,2 g (0,105 Mol) Diäthylchlorphosphat zu.

Separat werden 11,2 g (0,10 Mol) Kalium-t-butylat in 30 ml Dimethylformamid gelöst. Diese Lösung wird auf ca. −50° gekühlt und unter Argon mit 11,3 g (0,10 Mol) Isocyanessigsäureäthylester versetzt. Anschließend wird diese Lösung bei 18 bis 23° unter Kühlung zum obigen Reaktionsgemsich getropft. Man rührt während 1 Stunde bei Raumtemperatur, gibt 5 ml Essigsäure zu, gießt dann auf 500 ml Wasser und extrahiert zweimal mit je 200 ml Chloroform. Die vereinigten Chloroformauszüge werden dreimal mit je 300 ml Wasser gewaschen, über Magnesiumsulfat getrocknet und eingedampft. Zum öligen Rückstand gibt man 150 ml Essigester und läßt bei 0° kristallisieren. Man nutscht die ausgeschiedenen Kristalle ab, wäscht sie mit kaltem Essigester und erhält Äthyl-5,6-dihydro-5-methyl-6-oxo-4H-imidazo[1,5-a] [1,4]benzodiazepin-3-carboxylat vom Schmelzpunkt 163—165°. Nach Umkristallisieren aus Essigester zeigt das Produkt einen Schmelzpunkt von 164—165°.

b) 50 g (175,3 mMol) Äthyl-5,6-dihydro-5-methyl-6-oxo-4H-imidazo[1,5-a] [1,4]benzodiazepin-3-carboxylat in 400 ml Äthanol werden zusammen mit 150 ml Hydrazinhydrat während 2 Stunden unter Rückfluß zum Sieden erhitzt. Das ausgefallene Material wird abgenutscht und getrocknet. Man erhält 5,6-Dihydro-5-methyl-6-oxo-4H-imidazo[1,5-a] [1,4]benzodiazepin-3-carbonsäurehydrazid vom Schmelzpunkt 312—313°.

c) Eine Lösung von 19 g (70 mMol) 5,6-Dihydro-5-methyl-6-oxo-4H-imidazo[1,5-a] [1,4]benzodiazepin-3-carbonsäurehydrazid in 60 ml Wasser und 150 ml Eisessig wird bei 0° bis 5° tropfenweise mit einer Lösung von 16,5 g Natriumnitrit in 30 ml Waser versetzt. Die weiße Suspension wird anschließend während 1 Stunde im Eisbad gerührt und vorsichtig auf 150 g Natriumcarbonat in 400 ml Wasser gegossen. Das ausgefallene Material wird abgenutscht, mit Wasser gewaschen und getrocknet. Man erhält 5,6-Dihydro-5-methyl-6-oxo-4H-imidazo[1,5-a] [1,4]benzodiazepin-3-carbonsäureazid das sich bei 160° zersetzt.

d) 17 g 5,6-Dihydro-5-methyl-6-oxo-4H-imidazo[1,5-a] [1,4]benzodiazepin-3-carbonsäureazid werden mit 150 ml Benzylalkohol während 7 Stunden bei 110° gerührt. Die Lösung wird auf das halbe Volumen eingeengt und mit Äthanol verdünnt. Das ausgefallene Material wird abgenutscht, mit Äthanol gewaschen und getrocknet. Man erhält Benzyl-5,6-dihydro-5-methyl-6-oxo-4H-imidazo[1,5-a] [1,4]benzodiazepin-3-carbamat vom Schmelzpunkt 246—247°.

e) 34 g (93,8 mMol) Benzyl-5,6-dihydro-5-methyl-6-oxo-4H-imidazo[1,5-a] [1,4]benzodiazepin-3-carbamat werden in 300 ml Methanol in Gegenwart von 4 g 10proz. Palladium/Kohle bei Raumtemperatur und Normaldruck hydriert. Nach Entfernen des Katalysators und Eindampfen wird der Rückstand aus Essigester und Hexan umkristallisiert. Man erhält 3-Amino-4,5-dihydro-5-methyl-6H-imidazo[1,5-a] [1,4]benzodiazepin-6-on vom Schmelzpunkt 202—204°.

f) Eine Lösung von 5 g (22 mMol) 3-Amino-4,5-dihydro-5-methyl-6H-imidazo[1,5-a] [1,4]benzodiazepin-6-on in 100 ml Borfluorwasserstoffsäure (50proz. in Wasser) wird bei 0° bis 5° mit einer Lösung von 1,95 g (28 mMol) Natriumnitrit in 4 ml Wasser diazotiert. Die gelbe Lösung wird während 20 Minuten im Eisbad gerührt, mit 85 ml Borfluorwasserstoffsäure verdünnt, in ein Quartz-Gefäß umgegossen und über Nacht mit einer UV-Lampe bestrahlt. Anschließend extrahiert man mit Chloroform. Die Chloroformauszüge werden mit Wasser gewaschen, über Magnesiumsulfat getrocknet und eingedampft. Durch Chromatographieren des Rückstandes an einer Kieselgelsäule erhält man 3-Fluor-4,5-dihydro-5-methyl-6H-imidazo[1,5-a] [1,4]benzodiazepin-6-on vom Schmelzpunkt 171—172°.

Beispiel 11

Eine Lösung von 1,08 ml (40 mMol) 90proz. Wasserstoffperoxid in 20 ml Methylenchlorid versetzt man bei 5° bis 10° tropfenweise mit 6,80 ml (48 mMol) Trifluoressigsäureanhydrid und rührt während 10 Minuten im Eisbad. Man entfernt das Kühlbad und tropft über einen Zeitraum von 15 Minuten eine Suspension von 2,28 g (10 mMol) 3-Amino-4,5-dihydro-5-methyl-6H-imidazo[1,5-a] [1,4]benzodiazepin-6-on in 20 ml Methylenchlorid zu. Die Temperatur steigt bis zum Siedepunkt. Man rührt anschließend noch während 1 Stunde bei der Siedetemperatur. Das Reaktionsgemisch wird anschließend gekühlt und viermal mit Wasser und zweimal mit gesättigter Natriumbicarbonatlösung gewaschen. Die organische Phase wird über Magnesiumsulfat getrocknet und eingedampft. Durch Umkristallisieren aus Essigester erhält man 4,5-Dihydro-5-methyl-3-nitro-6H-imidazo[1,5-a] [1,4]benzodiazepin-6-on vom Schmelzpunkt 227—229°.

Beispiel 12

a) Eine Lösung von 14,26 g (0,05 Mol) Äthyl-5,6-dihydro-5-methyl-6-oxo-4H-imidazo[1,5-a] [1,4]benzodiazepin-3-carboxalat und 2,2 g (0,055 Mol) Nariumhydroxid in 100 ml Äthanol und 20 ml Wasser wird während 45 Minuten unter Rückfluß zum Sieden erhitzt und anschließend mit 55 ml 1 N-Salzsäure und 50 ml Wasser versetzt. Nach Abdestillieren des Äthanols wird der entstandene Kristallbrei abgenutscht, mit Wasser gewaschen und getrocknet. Man erhält 5,6-Dihydro-5-methyl-6-oxo-4H-imidazo[1,5-a] [1,4]benzodiazepin-3-carbonsäure vom Schmelzpunkt 287°.

b) 8,2 g 5,6-Dihydro-5-methyl-6-oxo-4H-imidazo[1,5-a] [1,4]benzodiazepin-3-carbonsäure werden auf 300° erhitzt. Nach der Beendigung der Kohlendioxidentwicklung läßt man erkalten und kristallisiert das Material aus Chloroform/Hexan um. Nach dem Trocknen erhält man 4,5-Dihydro-5-methyl-6H-imidazo[1,5-a] [1,4]benzodiazepin-6-on vom Schmelzpunkt 212—213°.

Beispiel 13

5 g (23,5 mMol) 4,5-Dihydro-5-methyl-6H-imidazo[1,5-a] [1,4]benzodiazepin-6-on in 30 ml Dimethylformamid werden mit 3,20 g (23,5 mMol) N-Chlorsuccinimid versetzt und während 1,5 Stunden bei Raumtemperatur und 10 Minuten bei 60° gerührt. Die braune, klare Lösung wird auf ca. 200 ml Wasser gegossen und dreimal mit Chloroform extrahiert. Die vereinigten Chloroformauszüge werden mit Wasser gewaschen, über Magnesiumsulfat getrocknet und eingedampft. Durch Chromatographieren des Rückstandes an einer Kieselgelsäule und anschließendes Umkristallisieren aus Essigester erhält man 3-Chlor-4,5-dihydro-5-methyl-6H-imidazo[1,5-a] [1,4]benzodiazepin-6-on vom Schmelzpunkt 193—194°.

Beispiel 14

20 g (93,8 mMol) 4,5-Dihydro-5-methyl-6H-imidazo[1,5-a] [1,4]benzodiazepin-6-on in 100 ml Dimethylformamid werden mit 17,5 g (98 mMol) N-Bromsuccinimid versetzt und 35 Minuten bei Raumtemperatur gerührt. Die Lösung wird auf Wasser gegossen und zweimal mit Chloroform extrahiert. Die Chloroformauszüge werden mehrmals mit Waser gewaschen, über Magnesiumsulfat getrocknet und eingedampft. Der Rückstand liefert nach Umkristallisieren aus Essigester 3-Brom-4,5-dihydro-5-methyl-6H-imidazo[1,5-a] [1,4]benzodiazepin-6-on vom Schmelzpunkt 196—198°.

Beispiel 15

1,06 g (5 mMol) 4,5-Dihydro-5-methyl-6H-imidazo[1,5-a] [1,4]benzodiazepin—6-on werden zusammen mit 1,26 g Jod in 20 ml Dimethylformamid während 40 Stunden bei 90° gerührt. Das Gemisch wird auf Wasser gegossen und dreimal mit Chloroform extrahiert. Die Chloroformauszüge werden fünfmal mit Wasser gewaschen, über Magnesiumsulfat getrocknet und eingedampft. Durch Chromatographieren des Rückstandes an einer Kieselgelsäule erhält man 4,5-Dihydro-3-jod-5-methyl-6H-imidazo[1,5-a] [1,4]benzodiazepin-6-on vom Schmelzpunkt 174—176°.

Beispiel 16

3,71 g (15 mMol) 3-Chlor-4,5-dihydro-5-methyl-6H-imidazo[1,5-a] [1,4]benzodiazepin-6-on in 10 ml Toluol werden zusammen mit 3,03 g (7,5 mMol) 2,4-Bis-(p-methoxyphenyl)-1,3,2,4-dithiadiphosphetan-2,4-disulfid während 4 Stunden unter Rückfluß zum Sieden erhitzt. Die Lösung wird mit Wasser gewaschen und eingedampft. Durch Chromatographieren an einer Kieselgelsäule und anschließendes Umkristallisieren aus Essigester erhält man 3-Chlor-4,5-dihydro-5-methyl-6H-imidazo[1,5-a] [1,4]benzodiazepin-6-thion vom Schmelzpunkt 197,5—198,5°.

Beispiel 17

a) Unter einer Argonatmosphäre werden 21,5 g (75,4 mMol) Äthyl,5,6-dihydro-5-methyl-6-oxo-4H-imidazo[1,5-a] [1,4]benzodiazepin-3-carboxylat unter Rühren in 250 ml absolutem Tetrahydrofuran (über Alox basisch I filtriert) heiß gelöst, auf ca. 30° abgekühlt und tropfenweise mit einer Lösung von 1,66 g (75,4 mMol) Lithiumborhydrid in 25 ml absolutem Tetrahydrofuran versetzt. Das Reaktionsgemisch wird 6 Stunden unter Rückfluß zum Sieden erhitzt, auf Raumtemperatur abgekühlt und mit 50 ml 3 N-wäßriger Salzsäure zersetzt. Man rührt weitere 2 Stunden bei 60° und entfernt das Tetrahydrofuran im Vakuum. Der Rückstand wird mit konz. Ammoniak alkalisch gestellt und 2 Stunden im Eisbad stehengelassen. Das Rohprodukt wird abgenutscht, mit viel Wasser gewaschen und aus Äthanol umkristallisiert. Man erhält 4,5-Dihydro-3-(hydroxymethyl)-5-methyl-6H-imidazo[1,5-a] [1,4]benzodiazepin-6-on vom Schmelzpunkt 224—226°.

b) 6 g (24,6 mMol) 4,5-Dihydro-3-(hydroxymethyl)-5-5-methyl-6H-imidazo[1,5-a] [1,4]benzodiazepin-6-on und 4,5 g Thionylchlorid werden während 2 Stunden in 50 ml Benzol unter Rückfluß zum Sieden erhitzt. Man dampft zur Trocknene ein, nimmt den Rückstand in Chloroform auf und wäscht mit 2 N-Natronlauge. Man trocknet die organische Phase über Magnesiumsulfat und erhält nach Entfernen des Lösungsmittels 3-(Chlormethyl)-4,5-dihydro-5-methyl-6H-imidazo[1,5-a] [1,4]benzodiazepin-6-on vom Schmelzpunkt 277—280°.

c) 1,30 g (5 mMol) 3-(Chlormethyl)-4,5-dihydro-5-methyl-6H-imidazo[1,5-a] [1,4]benzodiazepin-6-on und 200 mg (5 mMol) Natriumhydroxid werden in 20 ml Alkohol und in Gegenwart von 100 mg 10proz. Palladium/Kohle bei Raumtemperatur und Normaldruck hydriert. Der Katalysator wird entfernt, das Lösungsmittel eingedampft und der Rückstand in Chloroform aufgenommen. Man wäscht mit Wasser, trocknet über Magnesiumsulfat und dampf ein. Man erhält 4,5-Dihydro-3,5-dimethyl-6H-imidazo[1,5-a] [1,4]benzodiazepin-6-on vom Schmelzpunkt 184,5—185,5°.

Beispiel 18

a) Eine Lösung von 35 g (0,14 mMol) (S)-(+)-7-Chlor-1,2,3,11a-tetrahydro-5H-pyrrolo[2,1-c] [1,4]benzodiazepin-5,11(10H)-dion in 170 ml trockenem Dimethylformamid versetzt man unter einer Argonatmosphäre mit 17,3 g (0,15 Mol) Kalium-t-butylat, wobei die Temperatur von 24° auf 40° steigt. Man kühlt auf —30° und tropft, zwischen —30 und —20°, 25 g (0,15 Mol) Diäthylchlorphosphat zu.

Separat werden 16,8 g Kalium-t-butylat (0,15 Mol) in 50 ml Diemthylformamid gelöst. Diese Lösung wird auf ca. —50° gekühlt und unter Argon mit 17,42 g (0,15 Mol) Isocyanessigsäureäthylester versetzt. Anschließend wird diese Lösung bei —20 bis —10° zum obigen Reaktionsgemisch getropft. Man rührt während 1 Stunde ohne Kühlung, gibt 14 ml Essigsäure zu, gießt anschließend auf ca. 1000 ml Wasser und extrahiert dreimal mit je 250 ml Chloroform. Die vereinigten Chloroformphasen werden fünfmal mit je 300 ml Wasser gewaschen, über Magnesiumsulfat getrocknet und eingedampft. Der Rückstand wird aus 500 ml Essigester umkristallisiert. Man erhält (S)-(+)-Äthyl-7-chlor-11,12,13,13a-tetrahydro-9-oxo-9H-imidazo[1,5-a]pyrrolo[2,1-c] [1,4]benzodiazepin-1-carboxylat vom Schmelzpunkt 242—244°:

b) 7 g (20,2 mMol) (S)-(+)-Äthyl-7-chlor-11,12,13,13a-tetrahydro-9-oxo-9H-imidazo[1,5-a]pyrrolo[2,1-c] [1,4]benzodiazepin-1-carboxylat und 950 mg (23,8 mMol) Natriumhydroxid werden in 20 ml Alkohol und 4 ml Wasser während 30 Minuten unter Rückfluß zum Sieden erhitzt. Nach Abdampfen des Äthanols wird der Rückstand mit 23,7 ml 1 N-Salzsäure sauer gestellt. Das erhaltene weiße Material wird abgenutscht, mit wenig Wasser gewaschen und getrocknet. Man erhält (S)-7-Chlor-11,12,13,13a-tetrahydro-9-oxo-9H-imidazo[1,5-a]pyrrolo[2,1-c) [1,4]benzodiazepin-1-carbonsäure vom Schmelzpunkt 227°.

c) 5,8 g (18,2 mMol) (S)-7-Chlor-11,12,13,13a-tetrahydro-9-oxo-9H-imidazo[1,5-a]pyrrolo[2,1-c] [1,4]benzodiazepin-1-carbonsäure werden auf 230° erhitzt. Nach Beendigung der Kohlendioxidentwicklung wird die Schmelze mit Essigester behandelt. Das feste Material wird abgenutscht und getrocknet. Man erhält (S)-7-Chlor-11,12,13,13a-tetrahydro-9H-imidazo[1,5-a]pyrrolo[2,1-c] [1,4]benzodiazepin-9-on vom Schmelzpunkt 216—217°.

Beispiel 19

3 g (22 mMol) (S)-7-Chlor-11,12,13,13a-tetrahydro-9H-imidazo[1,5-a]pyrrolo[2,1-c] [1,4]benzodiazepin-9-on werden in 30 ml Dimethylformamid mit 1,60 g N-Chlorsuccinimid während 30 Minuten bei 90° gerührt. Die Lösung wird auf Wasser gegossen und mit Chloroform extrahiert. Die Chloroformauszüge werden mehrmals mit Wasser gewaschen, über Magnesiumsulfat getrocknet und eingedampft. Durch Chromatographieren an einer Kieselgelsäule und anschließendes Umkristallisieren aus Essigester erhält man (S)-1,7-Dichlor-11,12,13,13a-tetrahydro-9H-imidazo[1,5-a]pyrrolo[2,1-c] [1,4]benzodiazepin-9-on vom Schmelzpunkt 209—210°.

Beispiel 20

a) Unter einer Argonatmosphäre versetzt man eine Lösung von 21,6 g (0,10 Mol) (S)-(+)-1,2,3,11a-Tetrahydro-5H-pyrrolo[2,1-c) [1,4]benzodiazepin-5,11(10H)-dion in 100 ml trockenem Dimethylformamid mit 13,5 g (0,12 Mol) Kalium-t-butylat, wobei die Temperatur von 24° auf 46° steigt. Man kühlt auf Raumtemperatur und tropft zwischen 18 bis 23° 18,2 g (0,0105 Mol) Diäthylchlorphosphat zu.

Separat werden 11,2 g (0,10 Mol) Kalium-t-butylat in 30 ml Dimethylformamid gelöst. Diese Lösung wird auf ca. −50° gekühlt und unter Argon mit 11,3 g (0,10 Mol) Isocyanessigsäureäthylester versetzt. Anschließend wird diese Lösung bei 18 bis 23° unter Kühlung zum obigen Reaktionsgemisch getropft. Man rührt während 1 Stunde bei Raumtemperatur, gibt 5 ml Essigsäure zu, gießt dann auf 500 ml Wasser und extrahiert zweimal mit je 200 ml Chloroform. Die vereinigten Chloroformphasen werden dreimal mit je 300 ml Wasser gewaschen, über Magnesiumsulfat getrocknet und eingedampft. Zum öligen Rückstand gibt man 150 ml Essigsäure und läßt bei 0° kristallisieren. Man nutscht die ausgeschiedenen Kristalle ab, wäscht mit kaltem Essigester und erhält Äthyl-(S)-11,12,13,13a-tetrahydro-9-oxo-9H-imidazo[1,5-a]pyrrolo[2,1-c] [1,4]benzodiazepin-1-carboxylat vom Schmelzpunkt 196—197°. Die Mutterlauge wird eingedampft und der Rückstand wird in 50 ml Essigester gelöst. Daraus kristallisiert eine weitere Portion des obigen Produkts; Schmelzpunkt 195—196°.

b) Eine Lösung von 12,45 g (0,04 Mol) Äthyl-(S)-11,12,13,13a-tetrahydro-9-oxo-9H-imidazo[1,5-a]pyrrolo[2,1-c] [1,4]benzodiazepin-1-carboxylat und 1,6 g (0,04 Mol) Natriumhydroxid in 100 ml Äthanol und 25 ml Wasser wird während 30 Minuten unter Rückfluß zum Sieden erhitzt. Anschließend gibt man 40 ml 1 N-Salzsäure zu und dampft auf das halbe Volumen ein. Die dabei ausgefallenen Kristalle werden abgenutscht, mit Wasser gewaschen und getrocknet. Man erhält (S)-11,12,13,13a-Tetrahydro-9-oxo-9H-imidazo[1,5-a]pyrrolo[2,1-c] [1,4]benzodiazepin-1-carbonsäure vom Zersetzungspunkt 225°.

c) 8,8 g (S)-11,12,13,13a-Tetrahydro-9-oxo-9H-imidazo[1,5-a]pyrrolo[2,1-c] [1,4]benzodiazepin-1-carbonsäure werden auf 240° erhitzt. Nach Beendigung der Kohlendioxidentwicklung wird der Rückstand aus Essigester umkristallisiert. Man erhält (S)-11,12,13,13a-Tetrahydro-9H-imidazo[1,5-a]pyrrolo[2,1-c] [1,4]benzodiazepin-9-on vom Schmelzpunkt 211—212.

Beispiel 21

2,5 g (10,4 mMol) (S)-11,12,13,13a-Tetrahydro-9H-imidazo[1,5-a]pyrrolo[2,1-c] [1,4]benzodiazepin-9-on werden in 20 ml Dimethylformamid mit 1,40 g (10,4 mMol) N-Chlorsuccinimid während 1 Stunde bei Raumtemperatur und 20 Minuten bei 75° gerührt. Die Lösung wird auf Wasser gegossen und mit Chloroform extrahiert. Die Chloroformauszüge werden mehrmals mit Wasser gewaschen, über Magnesiumsulfat getrocknet und eingedampft. Durch Chromatographieren des Rückstandes an einer Kieselgelsäule erhält man (S)-1-Chlor-11,12,13,13a-tetrahydro-9H-imidazo[1,5-a]pyrrolo[2,1-c] [1,4]benzodiazepin-9-on vom Schmelzpunkt 233—234,5°.

Beispiel 22

2,5 g (10,4 mMol) (S)-11,12,13,13a-Tetrahydro-9H-imidazo[1,5-a]pyrrolo[2,1-c] [1,4]benzodiazepin-9-on werden in 20 ml Dimethylformamid mit 1,86 g N-Bromsuccinimid (10,4 mMol) während 30 Minuten bei Raumtemperatur gerührt. Die Lösung wird auf Wasser gegossen und mit Chloroform extrahiert. Die Chloroformauszüge werden mehrmals mit Wasser gewaschen, über Magnesiumsulfat getrocknet und eingedampft. Nach zweimaligem Umkristallisieren aus Essigester erhält man (S)-1-Brom-11,12,13,13a-tetrahydro-9H-imidazo[1,5-a]pyrrolo[2,1-c] [1,4]benzodiazepin-9-on vom Schmelzpunkt 225—226°.

Beispiel 23

Ein Gemisch aus 1,1 g (4,5 mMol) 4,5-Dihydro-3-(hydroxymethyl)-5-methyl-6H-imida-

zo[1,5-a] [1,4]benzodiazepin-6-on, 0,3 ml (5,0 mMol) Methyljodid und 10 ml trockenem Dimethylformamid wird mit 0,2 g (4,5 mMol) Natriumhydrid (55proz. Öldispersion, mit n-Hexan gewaschen) und während etwa 16 Stunden bei Raumtemperatur gerührt. Man gießt auf 70 ml Wasser und extrahiert dreimal mit Chloroform. Die Chloroformlösung wird einmal mit Wasser gewaschen, über Magnesiumsulfat getrocknet und eingedampft. Das Rohprodukt chromatographiert man an Kieselgel unter Eluieren mit Chloroform, das 5% Methanol enthält. Umkristallisieren des erhaltenen Materials aus Essigester/Hexan liefert 4,5-Dihydro-3-(methoxymethyl)-5-methyl-6H-imidazo[1,5-a] [1,4]benzodiazepin-6-on vom Schmelzpunkt 115—116°.

Beispiel 24

a) Zu einer heißen Lösung von 5,2 g (16,2 mMol) Äthyl-7-chlor-5,6-dihydro-5-methyl-6-oxo-4H-imidazo[1,5-a] [1,4]benzodiazepin-3-carboxylat in 180 ml trockenem Tetrahydrofuran tropft man eine Lösung von 0,39 g (17,9 mMol) Lithiumborhydrid in 40 ml trockenem Tetrahydrofuran. Man rührt während 4 Stunden bei Siedetemperatur und versetzt anschließend bei Raumtemperatur tropfenweise mit 10,4 ml 3 N-Salzsäure. Das erhaltene Gemisch wird während 1,5 Stunden unter Rückfluß zum Sieden erhitzt. Man entfernt das Tetrahydrofuran im Vakuum, versetzt den wäßrigen Rückstand mit konz. Ammoniak, bis das Gemisch basisch reagiert, und extrahiert dreimal mit Methylenchlorid. Die Methylenchloridlösung wird über Magnesiumsulfat getrocknet und im Vakuum eingedampft. Der kristalline Rückstand liefert nach Umkristallisieren aus Äthanol 7-Chlor-4,5-dihydro-3-(hydroxymethyl)-5-methyl-6H-imidazo[1,5-a] [1,4]benzodiazepin-6-on vom Schmelzpunkt 207—208°.

b) Ein Gemisch aus 2,22 g (8 mMol) 7-Chlor-4,5-dihydro-3-(hydroxymethyl)-5-methyl-6H-imidazo[1,5-a] [1,4]benzodiazepin-6-on, 0,6 ml (10 mMol) Methyljodid und 20 ml trockenem Dimethylformamid wird mit 0,4 g (9 mMol) Natriumhydrid (55proz. Öldisperison, mit n-Hexan gewaschen) versetzt und während 23 Stunden bei Raumtemperatur gerührt. Man gießt auf 50 ml Wasser und extrahhiert viermal mit Methylenchlorid. Die Methylenchloridlösung wird über Magnesiumsulfat getrocknet und eingedampft. Das Rohprodukt wird unter Eluieren mit Methylenchlorid, das 1% Methanol enthält, an Kieselgel chromatographiert. Durch anschließendes Umkristallisieren aus Essigester/n-Hexan erhält man 7-Chlor-4,4-dihydro-3-(methoxymethyl)-5-methyl-6H-imidazo[1,5-a] [1,4]benzodiazepin-6-on vom Schmelzpunkt 143—144°.

3,7-Dichlor-4,5-dihydro-5-methyl-6H-imidazo[1,5-a] [1,4]benzodiazepin-6-on (Wirkstoff A), (R,S)-1,8-Dichlor-11,13a-dihydro-9H-imidazo[1,5-a]pyrrolo[2,1-c] [1,4]-benzodiazepin-9-on (Wirkstoff B) und 3-Chlor-4,5-dihydro-5-methyl-6H-imidazo[1,5-a] [1,4]benzodiazepin-6-on (Wirkstoff C) können wie in den Beispielen A bis G angegeben als Wirkstoffe zur Herstellung von pharmazeutischen Präparaten verwendet werden:

Beispiel A

Es werden in üblicher Weise Tabletten folgender Zusammensetzung hergestellt:

| | mg/Tablette |
|---|---|
| Wirkstoff A, B oder C | 1 |
| Milchzucker | 103 |
| Maisstärke | 25 |
| Mikrokristalline Cellulose | 70 |
| Magnesiumstearat | 1 |
| Total | 200 |

Beispiel B

Es werden Kapseln folgender Zusammensetzung hergestellt:

| | mg/Kapsel |
|---|---|
| Wirkstoff A, B oder C | 1 |
| Milchzucker | 164 |
| Maisstärke | 30 |
| Talk | 5 |
| Total | 200 |

Der Wirkstoff, Milchzucker und Maisstärke werden zunächst in einem Mischer und dann in einer Zerkleinerungsmaschine vermengt. Man bringt das Gemisch wieder in den Mischer zurück, gibt den Talk zu und vermengt gründlich. Das Gemisch wird maschinell in Hartgelatinekapseln abgefüllt.

## Beispiel C

Es werden Injektionslösungen folgender Zusammensetzung hergestellt:

| | pro ml |
|---|---|
| Wirkstoff A, B oder C | 0,5 mg |
| Benzylalkohol | 0,015 ml |
| Propylenglykol | 0,4 ml |
| Äthanol (95proz.) | 0,1 ml |
| Natriumbenzoat | 48,8 mg |
| Benzoesäure | 1,2 mg |
| Wasser für Injektion q.s. ad | 1,0 ml |

Zur Herstellung von 10 000 ml Injektionslösung löst man 5 g des Wirkstoffes in 150 ml Benzylalkohol und gibt 4000 ml Propylenglykol und 1000 ml Äthanol zu. Dann löst man 12 g Benzoesäure in dem obigen Gemisch und gibt eine Lösung von 488 g Natriumbenzoat in 300 ml Wasser (für Injektion). zu. Die erhaltene Lösung wird durch Zugabe von Wasser (für Injektion) auf ein Volumen von 10 000 ml gebracht, filtriert und in Ampullen geeigneter Größe abgefüllt; man füllt das Restvolumen der Ampullen mit Stickstoff, schmilzt die Ampullen zu und sterilisiert sie während 30 Minuten im Autoklaven bei 0,7 atm.

## Beispiel D

Es werden Suppositorien folgender Zusammensetzung hergestellt:

| | g/Supp. |
|---|---|
| Wirkstoff A, B oder C | 0,001 |
| —Cacaobutter (Smp. 36—37°) | 1,255 |
| Carnauba-Wachs | 0,044 |
| Total | 1,3 |

Cacaobutter und Carnabau-Wachs werden in einem Glas- oder Stahlgefäß geschmolzen, gründlich vermengt und auf 45° abgekühlt. Hierauf gibt man den fein pulverisierten Wirkstoff zu und rührt, bis er vollständig dispergiert ist. Man gießt die Mischung in Suppositorienformen geeigneter Größe, läßt abkühlen, nimmt dann die Suppositorien aus den Formen und verpackt sie einzeln in Wachspapier oder Metallfolien.

## Beispiel E

Es werden Kapseln folgender Zusammensetzung hergestellt:

| | mg/Kapseln |
|---|---|
| Wirkstoff A, B oder C | 20,0 |
| (+)-5-(o-Chlorphenyl)-1,3-dihydro-3-methyl-7-nitro-2H-1,4-benzodiazepin-2-on (Wirkstoff D) | 30,0 |
| Milchzucker krist. | 100,0 |
| Maisstärke weiß | 27,5 |
| Talk | 10,0 |
| Magnesiumstearat | 2,5 |
| Total | 190,0 |

Die beiden Wirkstoffe werden mit den Hilfsstoffen gut vermischt und zu je 190,0 mg in Steckkapseln geeigneter Größe abgefüllt.

Beispiel F

Es werden Tabletten folgender Zusammensetzung hergestellt:

| | mg/Tablette |
|---|---|
| Wirkstoff A, B oder C | 10,0 |
| (+)-5-(o-Chlorphenyl)-1,3-dihydro-3-methyl-7-nitro-2H-1,4-benzodiazepin-2-on (Wirkstoff D) | 30,0 |
| Milchzucker pulvis | 15,0 |
| Maisstärke weiß | 19,5 |
| Povidon K30 | 3,5 |
| Maisstärke weiß | 10,0 |
| Magnesiumstearat | 2,0 |
| Total | 90,0 |

Die beiden Wirkstoffe, Milchzucker pulvis und die erste Portion Maisstärke weiß werden gemischt und gesiebt. Diese Mischung wird mit einer Lösung von Povidon K30 in Wasser befeuchtet, geknetet, granuliert, getrocknet und gesiebt. Dem Granulat werden die zweite Portion Maisstärke weiß und Magnesiumstearat zugesetzt. Nach Mischen wird die erhaltene Masse zu 90 mg schweren Tabletten verpreßt.

Beispiel G

Es werden Tabletten folgender Zusammensetzung hergestellt:

| | mg/Tablette |
|---|---|
| Wirkstoff A, B oder C | 30 |
| (+)-5-(o-Chlorphenyl)-1,3-dihydro-3-methyl-7-nitro-2H-1,4-benzodiazepin-2-on (Wirkstoff D) | 30 |
| Milchzucker pulvis | 22 |
| Maisstärke weiß | 22 |
| Povidon K30 | 6 |
| Maisstärke weiß | 16 |
| Magnesiumstearat | 4 |
| Total | 130 |

Die beiden Wirkstoffe, Milchzucker pulvis und die erste Portion Maisstärke weiß werden gemischt und gesiebt. Diese Mischung wird mit einer Lösung von Povidon K30 in Wasser befeuchtet, geknetet, granuliert, getrocknet und gesiebt. Dem Granulat werden die zweite Portion Maisstärke weiß und Magnesiumstearat zugesetzt. Nach Mischen wird die erhaltene Masse zu 130 mg schweren Tabletten verpreßt.

**Patentansprüche**

1. Imidazodiazepine der allgemeinen Formel

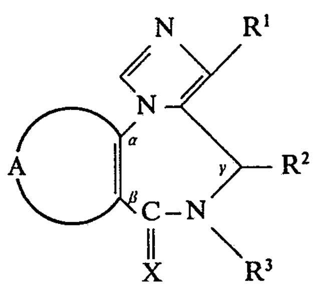

(I)

worin A zusammen mit den beiden mit $\alpha$ und $\beta$ bezeichneten Kohlenstoffatomen die Gruppe

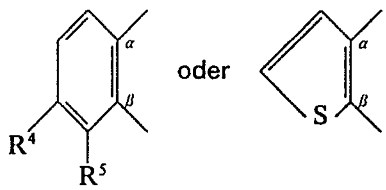

(a) oder (b)

$R^1$ Wasserstoff, $(C_{1-7})$-Alkyl, $(C_{1-7})$-Alkoxymethyl, Halogen oder Nitro, $R^4$ Wasserstoff, Trifluormethyl oder Halogen, $R^5$ Wasserstoff, Trifluormethyl, Halogen oder $(C_{1-7})$-Alkyl und X ein Sauerstoff- oder Schwefelatom bedeuten, und entweder $R^2$ Wasserstoff und $R^3$ $(C_{1-7})$-Alkyl bedeuten, oder $R^2$ und $R^3$ zusammen Trimethylen oder Propenylen bedeuten und das mit $\gamma$ bezeichnete Kohlenstoffatom die (S)- oder (R,S)-Konfiguration aufweist,
und pharmazeutisch annehmbare Säureadditionssalze davon.

2. Verbindungen gemäß Anspruch 1, dadurch gekennzeichnet, daß $R^1$ Wasserstoff, Methyl, Methoxymethyl, Halogen oder Nitro bedeutet.

3. Verbindungen gemäß Anspruch 1 oder 2, dadurch gekennzeichnet, daß $R^3$ Methyl bedeutet, wenn $R^2$ Wasserstoff bedeutet.

4. Verbindungen gemäß Anspruch 1 oder 2, dadurch gekennzeichnet, daß das in Formel I mit $\gamma$ bezeichnete Kohlenstoffatom die (S)-Konfiguration aufweist, wenn $R^2$ und $R^3$ zusammen Trimethylen bedeuten.

5. Verbindungen gemäß einem der Ansprüche 1 bis 4, dadurch gekennzeichnet, daß $R^4$ Wasserstoff, Chlor oder Fluor und $R^5$ Wasserstoff oder Chlor bedeuten, wobei mindestens eines von $R^4$ und $R^5$ Wasserstoff bedeutet, wenn A zusammen mit den beiden mit $\alpha$ und $\beta$ bezeichenten Kohlenstoffatomen die in Anspruch 1 definierte Gruppe (a) bedeutet.

6. 3,7-Dichlor-4,5-dihydro-5-methyl-6H-imidazo[1,5-a] [1,4]benzodiazepin-6-on.

7. (R,S)-1,8-Dichlor,11,13a-dihydro-9H-imidazo[1,5-a]pyrrolo[2,1-c] [1,4]benzodiazepin-9-on.

8. 3-Chlor-4,5-dihydro-5-methyl-6H-imidazo[1,5-a] [1,4]benzodiazepin-6-on.

9. 7-Chlor-4,5-dihydro-3-(methoxymethyl)-5-methyl-6H-imidazo[1,5-a] [1,4]benzodiazepin-6-on.

10. (S)-1-Chlor-10,11,12,12a-tetrahydro-8H-imidazo[5,1-c]pyrrolo[1,2-a]-thieno[3,2-e] [1,4]diazepin-8-on,
3-Chlor-8-fluor-4,5-dihydro-5-methyl-6H-imidazo[1,5-a] [1,4]benzodiazepin-6-on,
4,5-Dihydro-3-jod-5-methyl-6H-imidazo[1,5-a] [1,4]benzodiazepin-6-on,
3-Brom-4,5-dihydro-5-methyl-6H-imidazo[1,5-a] [1,4]benzodiazepin-6-on,
3-Brom-8-fluor-4,5-dihydro-5-methyl-6H-imidazo[1,5-a] [1,4]benzodiazepin-6-on,
3-Chlor-4,5-dihydro-5-methyl-6H-imidazo[1,5-a] [1,4]benzodiazepin-6-thion,
(S)-1-Chlor-11,12,13,13a-tetrahydro-9H-imidazo[1,5-a]pyrrolo[2,1-c] [1,4]benzodiazepin-9-on und
(S)-1-Brom-11,12,13,13a-tetrahydro-9H-imidazo[1,5-a]pyrrolo[2,1-c] [1,4]benzodiazepin-9-on.

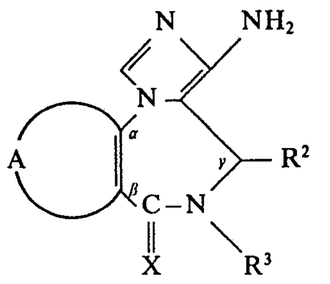

(III)

worin A, X, $R^2$ und $R^3$ die in Anspruch 1 angegebene Bedeutung besitzen.

12. Verbindungen der allgemeinen Formel

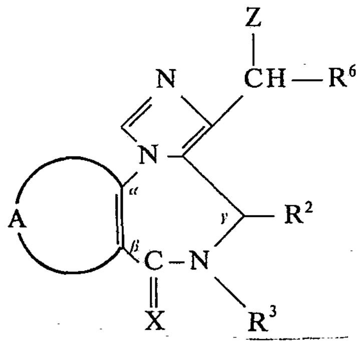

(IV)

worin A, X, $R^2$ und $R^3$ die in Anspruch 1 angegebene Bedeutung besitzen, Z eine Abgangsgruppe und $R^6$ Wasserstoff oder ($C_{1-6}$)-Alkyl bedeuten.

13. Verbindungen gemäß einem der Ansprüche 1 bis 10 als pharmazeutische Wirkstoffe.

14. Pharmazeutische Wirkstoffe gemäß Anspruch 13, welche die zentral dämpfenden, muskelrelaxierenden, ataktischen, blutdrucksenkenden und atemdepressiven Eigenschaften von tranquilisierend wirksamen 1,4-Benzodiazepinen antagonisieren.

15. Verfahren zur Herstellung von Verbindungen gemäß einem der Ansprüche 1 bis 10, dadurch gekennzeichnet, daß man

a) eine Verbindung der allgemeinen Formel

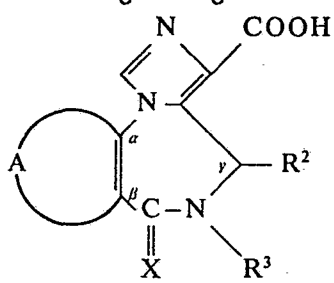

(II)

worin A, X, $R^2$ und $R^3$ die in Anspruch 1 angegebene Bedeutung besitzen, decarboxyliert, oder

b) eine Verbindung der allgemeinen Formel

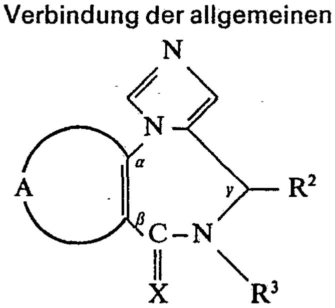

(Ia)

worin A, X, $R^2$ und $R^3$ die in Anspruch 1 angegebene Bedeutung besitzen, am Imidazolring halogeniert, oder

c) in einer Aminoverbindung der allgemeinen Formel

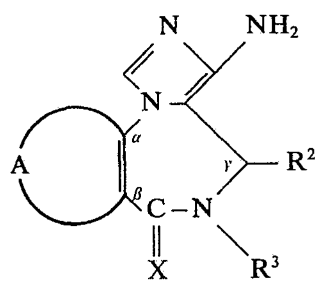

(III)

worin A, X, $R^2$ und $R^3$ die in Anspruch 1 angegebene Bedeutung besitzen, die Aminogruppe durch eine Nitrogruppe oder durch ein Halogenatom ersetzt, oder

d) in einer Aminoverbindung der obigen allgemeinen Formel III die Aminogruppe zur Nitrogruppe oxidiert, oder

e) eine Verbindung der allgemeinen Formel

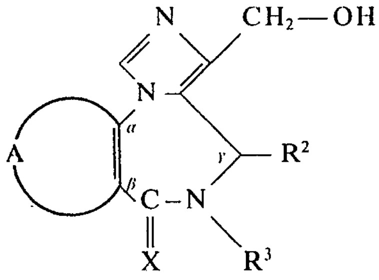

(X)

bzw.

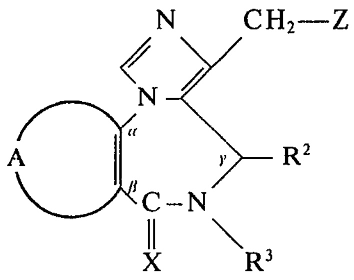

(IVa)

worin A, X, $R^2$ und $R^3$ die in Anspruch 1 angegebene Bedeutung besitzen und Z eine Abgangsgruppe bedeutet,
mit einem einen ($C_{1-7}$)-Alkylrest liefernden Alkylierungsmittel bzw. mit einem ($C_{1-7}$)-Alkohol veräthert, oder

f) in einer Verbindung der allgemeinen Formel

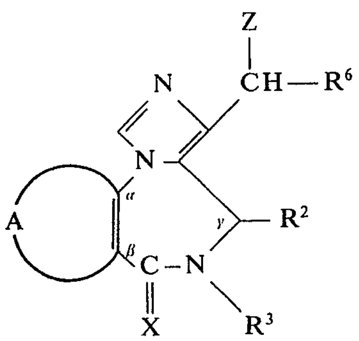

(IV)

worin A, X, $R^2$ und $R^3$ die in Anspruch 1 angegebene und Z obige Bedeutung besitzen und $R^6$ Wasserstoff oder ($C_{1-7}$)-Alkyl bedeutet,
die mit Z bezeichnete Abgangsgruppe unter reduktiven Bedingungen abspaltet, oder

g) in einer Verbindung der allgemeinen Formel

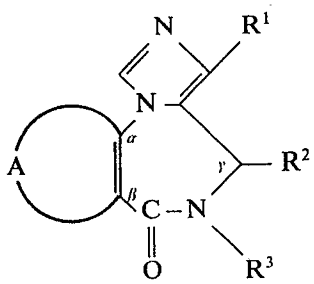

(Ib)

worin A, $R^1$, $R^2$ und $R^3$ die in Anspruch 1 angegebene Bedeutung besitzen,
die Carbonylgruppe in die Thiocarbonylgruppe überführt, und

h) erwünschtenfalls eine erhaltene Verbindung der allgemeinen Formel I in ein pharmazeutisch annehmbares Säureadditionssalz überführt.

16. Arzneimittel, enthaltend eine Verbindung gemäß einem der Ansprüche 1 bis 10.

17. Arzneimittel gemäß Anspruch 10, welche die zentral dämpfenden, muskelrelaxierenden, ataktischen, blutdrucksenkenden und atemdepressiven Eigenschaften von tranquilisierend wirksamen 1,4-Benzodiazepinen antagonisieren.

18. Schistosomizid wirksames Arzneimittel, enthaltend eine Verbindung gemäß einem der Ansprüche 1 bis 10 und (+)-5-(o-Chlorphenyl)-1,3-dihydro-3-methyl-7-nitro-2H-1,4-benzodiazepin-2-on.

## Claims

1. Imidazodiazepines of the general formula

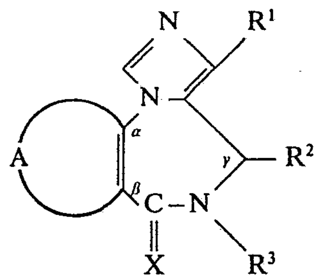

(I)

wherein A together with the two carbon atoms denoted as $\alpha$ and $\beta$ signifies the group

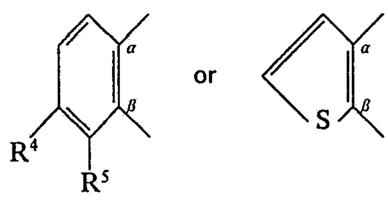

(a) or (b)

$R^1$ signifies hydrogen, $(C_{1-7})$-alkyl, $(C_{1-7})$alkoxymethyl, halogen or nitro, $R^4$ signifies hydrogen, trifluoromethyl or halogen, $R^5$ signifies hydrogen, trifluoromethyl, halogen or $(C_{1-7})$-alkyl and X signifies an oxygen or sulphur atom, and either $R^2$ signifies hydrogen and $R^3$ signifies $(C_{1-7})$-alkyl, or $R^2$ and $R^3$ together signify trimethylene or propenylene and the carbon atom denoted as $\gamma$ has the (S)- or (R,S)-configuration,
and pharmaceutically acceptable acid additions salts thereof.

2. Compounds according to claim 1, characterized in that $R^1$ signifies hydrogen, methyl, methoxymethyl, halogen or nitro.

3. Compounds according to claim 1 or 2, characterized in that $R^3$ signifes methyl when $R^2$ signifies hydrogen.

4. Compounds according to claim 1 or 2, characterized in that the carbon atom denoted as $\gamma$ in formula I has the (S)-configuration when $R^2$ and $R^3$ togehter signify trimethylene.

5. Compounds according to any one of claims 1 to 4, characterized in that $R^4$ signifes hydrogen, chlorine or fluorine and $R^5$ signifes hydrogen or chlorine, whereby at least one of $R^4$ and $R^5$ signifies hydrogen when A toghether with the two carbon atoms denoted as $\alpha$ and $\beta$ signifes the group (a) defined in claim 1.

6. 3,7-Dichloro-4,5-dihydro-5-methyl-6H-imidazo[1,5-a] [1,4]benzodiazepin-6-one.

7. (R,S)-1,8-Dichloro-11,13a-dihydro-9H-imidazo[1,5-a]pyrrolo[2,1-c] [1,4]benzodiazepin-9-one.

8. 3-Chloro-4,5-dihydro-5-methyl-6H-imidazo[1,5-a] [1,4]benzodiazepin-6-one.

9. 7-Chloro-4,5-dihydro-3-(methoxymethyl)-5-methyl-6H-imidazo[1,5-a] [1,4]benzodiazepin-6-one.

10. (S)-1-Chloro-10,11,12,12a-tetrahydro-8H-imidazo[5,1-c]pyrrolo[1,2-a]-thieno[3,2-e] [1,4]diazepin-8-one,
3-chloro-8-fluoro-4,5-dihydro-5-methyl-6H-imidazo[1,5-a] [1,4]benzodiazepin-6-one,
4,5-dihydro-3-iodo-5-methyl-6H-imidazo[1,5-a] [1,4]benzodiazepin-6-one,
3-bromo-4,5-dihydro-5-methyl-6H-imidazo[1,5-a] [1,4]benzodiazepin-6-one,
3-bromo-8-fluoro-4,5-dihydro-5-methyl-6H-imidazo[1,5-a] [1,4]benzodiazepin-6-one,
3-chloro-4,5-dihydro-5-methyl-6H-imidazo[1,5-a] [1,4]benzodiazepine-6-thione,
(S)-1-chloro-11,12,13,13a-tetrahydro-9H-imidazo[1,5-a]pyrrolo[2,1-c] [1,4)benzodiazepin-9-one and
(S)-1-bromo-11,12,13,13a-tetrahydro-9H-imidazo[1,5-a)pyrrolo[2,1,c] [1,4]benzodiazepin-9-one.

11. Compounds of the general formula

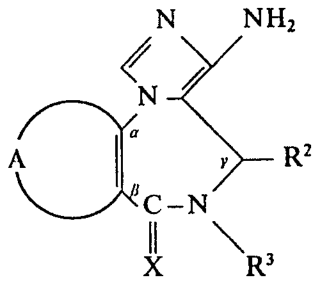

(III)

wherein A, X, $R^2$ and $R^3$ have the significance given in claim 1.

12. Compounds of the general formula

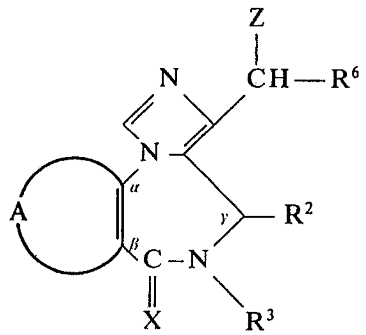

(IV)

wherein A, X, $R^2$ and $R^3$ have the significance given in claim 1, Z signifies a leaving group and $R^6$ signifies hydrogen or $(C_{1-6})$-alkyl.

13. Compounds according to any one of claims 1 to 10 as pharmaceutically active substances.

14. Pharmaceutically active substances according to claim 13, which antagonize the central-depressant, muscle relaxant, ataxic, blood pressure-lowering and respiratory-depressant properties of 1,4-benzodiazepines which have tranquillizing activity.

15. A process for the manufacture of compounds according to any one of claims 1 to 10, characterized by

a) decarboxylating a compound of the general formula

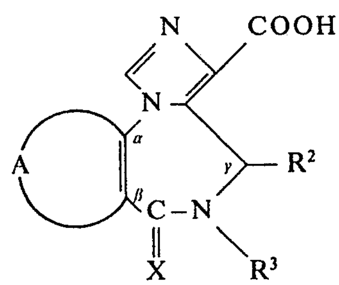

(II)

wherein A, X, $R^2$ and $R^3$ have the significance given in claim 1.

b) halogenating a compound of the general formula

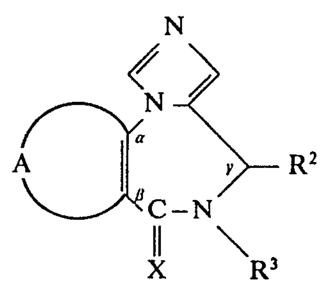

(Ia)

wherein A, X, $R^2$ and $R^3$ have the significance given in claim 1, on the imidazole ring, or

c) replacing the amino group in an amino compound of the general formula

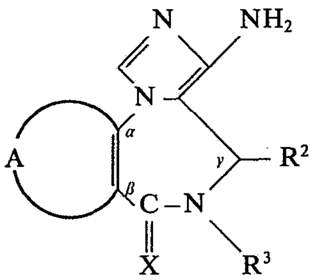

(III)

wherein A, X, $R^2$ and $R^3$ have the significance given in claim 1, by a nitro group or by a halogen atom, or

d) oxidizing the amino group in an amino compound of general formula III above to the nitro group, or

e) etherifying a compound of the general formula

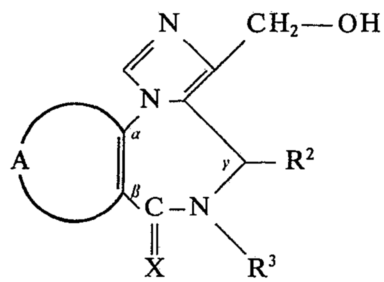

(X)

or

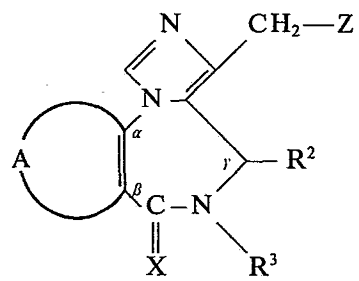

(IVa)

wherein A, X, $R^2$ and $R^3$ have the significance given in claim 1 and Z signifies a leaving group, with an alkylating agent yielding a $(C_{1-7})$-alkyl residue and with a $(C_{1-7})$-alcohol, respectively, or

f) cleaving off under reductive conditions the leaving group denoted by Z in a compound of the general formula

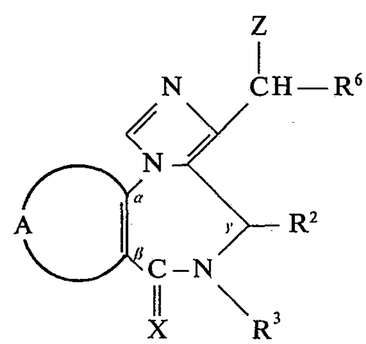

(IV)

wherein A, X, $R^2$ and $R^3$ have the significance given in claim 1 and Z has the above significance and $R^6$ signifies hydrogen or $(C_{1-6})$-alkyl,
or

g) converting the carbonyl group in a compound of the general formula

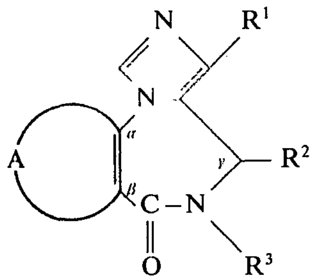

(Ib)

wherein A, $R^1$, $R^2$ and $R^3$ have the significance given in claim 1, into the thiocarbonyl group, and

h) if desired, converting a compound of general formula I obtained into a pharmaceutically acceptable acid addition salt.

16. A medicament containing a compound according to any one of claims 1 to 10.

17. A medicament according to claim 10, which antagonizes the central-depressant, muscle relaxant, ataxic, blood pressure-lowering and respiratory-depressant properties of 1,4-benzodiazepines which have tranquillizing activity.

18. A schistosomicidally-active medicament containing a compound according to any one of claims 1 to 10 and (+)-5-(o-chlorophenyl)-1,3-dihydro-3-methyl-7-nitro-2H-1,4-benzodiazepin-2-one.

**Revendications**

1. Imidazodiazépines de formule générale

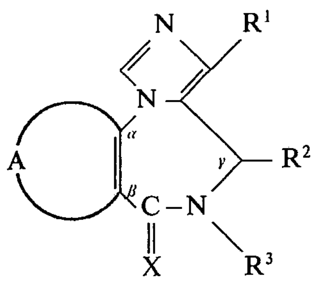

(I)

dans laquelle A forme avec les deux atomes de carbone marqués $\alpha$ et $\beta$ le groupe

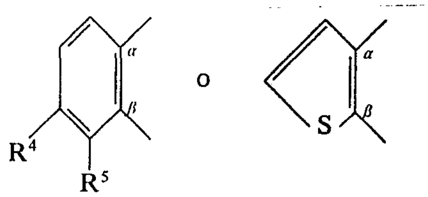

(a) o (b)

$R^1$ représente l'hydrogène, un groupe ($C_1$ à $C_7$)-alkyle, ($C_1$ à $C_7$)-alcoxyméthyle, un halogène ou un groupe nitro, $R^4$ représente l'hydrogène, un groupe trifluorométhyle ou un halogène, $R^5$ représente l'hydrogène, un groupe trifluorométhyle, un halogéne ou un groupe ($C_1$ à $C_7$)-alkyle et X représente un atome d'oxygène ou de soufre, et ou bien $R^2$ représente l'hydrogène et $R^3$ un groupe ($C_1$ à $C_7$)-alkyle, ou bien $R^2$ et $R^3$ forment ensemble un groupe triméthylène ou propénylène, et l'atome de carbone marqué $\gamma$ présente la configuration (S) ou (R,S), et leurs sels formés par addition avec des acides acceptables pour l'usage pharmaceutique,

2. Composés selon la revendication 1, caractérisés en ce que $R^1$ représente l'hydrogène, un groupe méthyle, méthoxyméthyle, un halogène ou le groupe nitro.

3. Composés selon la revendication 1 ou 2, caractérisés en ce que $R^3$ représente le groupe méthyle lorsque $R^2$ représente l'hydrogène.

4. Composés selon la revendication 1 ou 2, caractérisés en ce que l'atome de carbone marqué $\gamma$ dans la formule I présente la configuration (S) lorsque $R^2$ et $R^3$ forment ensemble le groupe triméthylène.

5. Composés selon l'une des revendications 1 à 4, caractérisés en ce que $R^4$ représente l'hydrogène, le chlore ou le fluor et $R^5$ représente l'hydrogène ou le chlore, l'un au moins des symboles $R^4$ et $R^5$

représentant l'hydrogène, lorsque A forme avec les deux atomes de carbone marqués $\alpha$ et $\beta$ le groupe (a) défine dans la revendication 1.

6. La 3,7-dichloro-4,5-dihydro-5-méthyl-6H-imidazo(1,5-a] [1,4]benzodiazépine-6-one.

7. La (R,S)-1,8-dichloro-11,13a-dihydro-9H-imidazo[1,5-a]pyrrrolo[2,1-c] [1,4]benzodiazépine-9-one.

8. La 3-chloro-4,5-dihydro-5-méthyl-6H-imidazo[1,5-a] [1,4]benzodiazépine-6-one.

9. La 7-chloro-4,5-dihydro-3-(méthoxyméthyl)-5-méthyl-6H-imidazo[1,5-a] [1,4]benzodiazépine-6-one.

10. La (S)-1-chloro-10,11,12,12a-tétrahydro-8H-imidazo[5,1-c]pyrrolo[1,2-a] thiéno[3,2-e] [1,4]diazépine-8-one,
la 3-chloro-8-fluoro-4,5-dihydro-5-méthyl-6H-imidazo[1,5-a] [1,4]benzodiazépine-6-one,
la 4,5-dihydro-3-iodo-5-méthyl-6H-imidazo[1,5-a] [1,4]benzodiazépine-6-one,
la 3-bromo-4,5-dihydro-5-méthyl-6H-imidazo[1,5-a] [1,4]benzodiazépine-6-one,
la 3-bromo-8-fluoro-4,5-dihydro-5-méthyl-6H-imidazo[1,5-a] [1,4]benzodiazépine-6-one,
la 3-chloro-4,5-dihydro-5-méthyl-6H-imidazo[1,5-a] [1,4]benzodiazépine-6-thione,
la (S)-1-chloro-11,12,13,13a-tétrahydro-9H-imidazo[1,5-a]pyrrolo[2,1-c] [1,4]benzodiazépine-9-one et
la (S)-1-bromo-11,12,13,13a-tétrahydro-9H-imidazo[1,5-a]pyrrolo[2,1-c] [1,4]benzodiazépine-9-one.

11. Composés de formule générale III

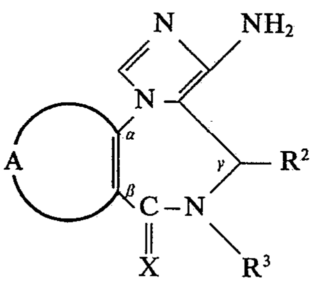

(III)

dans laquelle A, X, $R^2$ et $R^3$ ont les significations données à la revendication 1.

12. Composés de formule générale IV

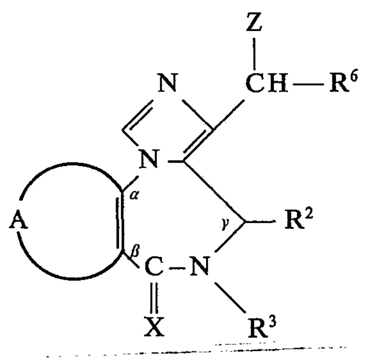

(IV)

dans laquelle A, X, $R^2$ et $R^3$ ont les significations donnés à la revendication 1 et Z représente un groupe éliminable et $R^6$ représente l'hydrogène ou un groupe ($C_1$ à $C_6$)-alkyle.

13. Composés selon l'une des revendications 1 à 10 en tant que substances actives pharmaceutiques.

14. Substances actives pharmaceutiques selon la revendication 13, qui antagonisent les effets de sédation centrale, de relaxation musculaire, d'ataxie, d'hypotension et de dépression respiratoire des 1,4-benzodiazépines à effet tranquillisant.

15. Procédé de préparation des composés selon l'une des revendications 1 à 10, caractérisé en ce que:

(a) on soumet à décarboxylation un composé de formule générale:

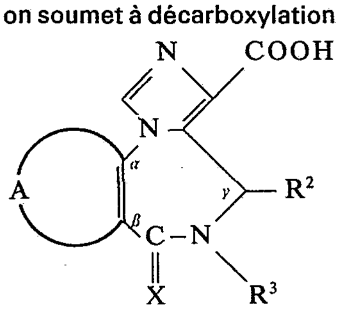

(II)

dans laquelle A, X, $R^2$ et $R^3$ ont les significations indiquées dans la revendication 1, ou bien

(b) on halogène sur le noyau imidazole un composé de formule générale

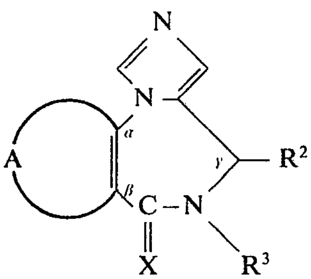

(Ia)

dans laquelle A, X, $R^2$ et $R^3$ ont les significations indiquées dans la revendication 1, ou bien

(c) dans un composé aminé de formule générale

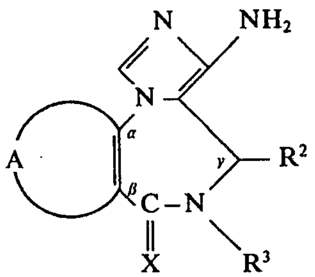

(III)

dans laquelle A, X, $R^2$ et $R^3$ ont les significations indiquées dans la revendications 1, on remplace le groupe amino par un groupe nitro ou par un atome d'halogène, ou bien

(d) dans un composé aminé de formule générale III ci-dessus, on oxyde le groupe amino en groupe nitro, ou bien

(e) on éthérifie un composé de formule générale respective:

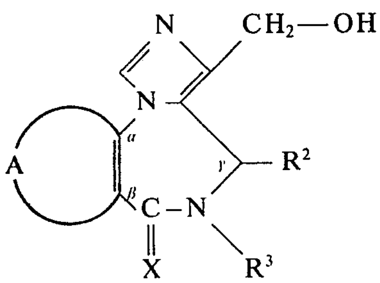

(X)

ou

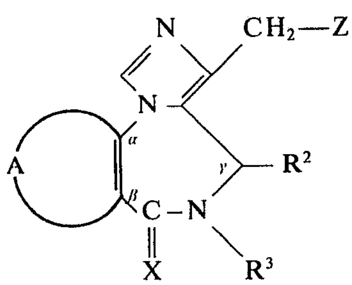

(IVa)

dans laquelle A, X, $R^2$ et $R^3$ ont les significations indiquées dans la revendication 1 et Z représente un groupe éliminable a l'aide respectivement d'un agent alkylant fournissant un groupe ($C_1$ à $C_7$)-alkyle ou à l'aide d'un alcool en $C_1$ à $C_7$, ou bien

(f) dans un composé de formule générale

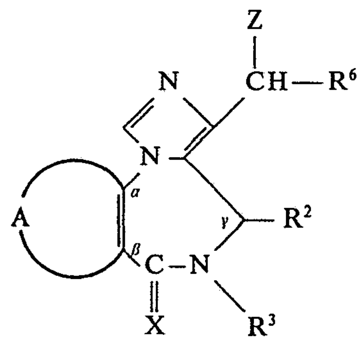

(IV)

dans laquelle A, X, $R^2$ et $R^3$ ont les significations indiquées dans la revendication 1 et 7 a la signification indiquée ci-dessus, $R^6$ représentant l'hydrogène ou un groupe ($C_1$ à $C_6$)-alkyle, on élimine le groupe éliminable représenté par Z dans des conditions réductrices, ou bien

(g) dans un composé de formule générale:

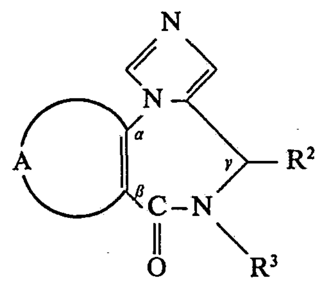

(Ib)

dans laquelle A, $R^1$, $R^2$ et $R^3$ ont les significations indiquées dans la revendication 1, on convertit le groupe carbonyle en groupe thiocarbonyle, et

(h) si on le désire, on convertit un composé obtenu de formule générale I en un sel formé par addition avec un acide acceptable pour l'usage pharmaceutique.

16. Médicaments contenant un composé selon l'une des revendications 1 à 10.

17. Médicaments selon la revendication 10, qui antagonisent les propriétés de sédation centrale, de relaxation musculaire, d'ataxie, d'hypotension et de dépression respiratoire des 1,4-benzodiazépines à effet tranquillisant.

18. Médicament à activité schistosomicide, contentant un composé selon l'une des revendications 1 à 10 et de la (+)-5-(o-chlorophényl)-1,3-dihydro-3-méthyl-7-nitro-2H-1,4-benzodiazépine-2-one.